# EUROPEAN PATENT APPLICATION

(11) **EP 4 417 684 A1**
(43) Date of publication of application: **21.08.2024**
(21) Application number: 22881009.9
(22) Date of filing: 11.10.2022
(51) Int. Cl.: C12N 1/21, A61K 35/74, A61K 35/742, A61K 35/744, A61K 35/745, A61K 35/747, A61P 1/00, A61P 1/02, A61P 3/04, A61P 9/10, A61P 17/00, A61P 17/02, A61P 35/00, C12N 15/12, C12P 1/04, C12P 21/02

(54) **BACTERIUM MODIFIED TO EXPRESS HETEROLOGOUS TAT PROTEIN**

(30) Priority: 11.10.2021 JP 2021167024
(71) Applicant: AJINOMOTO CO., INC., Chuo-ku Tokyo 104-8315 (JP)
(72) Inventor: USAMI Riku, Kawasaki-shi, Kanagawa 210-8681 (JP); TOYAZAKI Miku, Kawasaki-shi, Kanagawa 210-8681 (JP); NAKAMURA Hidehiro, Kawasaki-shi, Kanagawa 210-8681 (JP); MATSUDA Yoshihiko, Kawasaki-shi, Kanagawa 210-8681 (JP)
(74) Representative: Hoffmann Eitle
(86) International application number: PCT/JP2022/037882
(87) International publication number: WO 2023/063314

(57) **Abstract**

Provided is a technology whereby a heterologous protein can be expressed in a bacterium which does not have an endogenous Tat system secretion machine. The present specification discloses a bacterium that is selected from the group consisting of bifidobacteria, lactococci, and staphylococci and has been modified to express a heterologous Tat protein. The bacterium has the ability to produce and secrete a heterologous protein.

## Description

### Technical Field

The present invention relates to a bacterium modified to express heterologous Tat proteins. More specifically, the present invention relates to a bacterium selected from the group consisting of the genus Bifidobacterium, the genus Lactococcus, the genus Lactobacillus, the genus Limosilactobacillus, and the genus Staphylococcus, which has been modified to express a heterologous Tat protein.

### Background Art

A common production and secretion method for heterologous proteins involves utilizing a pathway known as the Sec system, which is widely present from prokaryotes to eukaryotes. In the Sec system, the proteins produced are extracellularly secreted in an unfolded state (not forming a higher-order structure).

Recently, a protein secretion pathway completely different from the Sec system has been discovered in the thylakoid membrane of plant cell chloroplasts (Non Patent Literature 1). Proteins secreted through this pathway share a common signal sequence featuring a sequence of arginine-arginine, which led to it being termed the Tat system (Twin-Arginine Translocation system). It is known that while in the Sec system pathway, proteins are secreted in a state before forming a higher-order structure as described above, in the Tat system pathway, proteins form a higher-order structure within the cell and then pass through the cell membrane and are secreted (Non Patent Literature 2).

A method for expressing heterologous proteins utilizing the Tat system is described in Patent Literature 1, where a construct including the tatABC gene derived from Streptomyces coelicolor, a nucleic acid sequence encoding a Tat signal peptide derived from S. scabies, and a nucleic acid sequence encoding agarase derived from Streptomyces coelicolor is introduced into Bacillus subtilis, achieving the expression of agarase derived from Streptomyces coelicolor within Bacillus subtilis.

Patent Literature 2 describes the introduction of a genetic construct including a nucleic acid sequence encoding a Tat system-dependent signal peptide and a nucleic acid sequence encoding a heterologous protein into a coryneform bacterium (Corynebacterim glutamicum) having a Tat secretion apparatus, enabling the production and secretion of the heterologous protein within the coryneform bacterium.

Non Patent Literature 3 describes the expression of Tat derived from Pseudomonas syringae, Streptomyces coelicolor, and Aquifex aeolicus within E. coli.

Non Patent Literature 4 describes the expression of Tat derived from Bacillus subtilis within E. coli.

### Citation List

### Patent Literatures

Patent Literature 1: WO2011/135370
Patent Literature 2: WO2005/103278

### Non Patent Literatures

Non Patent Literature 1: Chaddock AM, Mant A, Karnauchov I, Brink S, Herrmann RG, Klosgen RB, Robinson C, A new type of signal peptide: central role of a twin-arginine motif in transfer signals for the delta pH-dependent thylakoidal protein translocase, EMBO Journal, (1995), 14(12):2715-2722
Non Patent Literature 2: Hynds PJ, Robinson D, Robinson C. The sec-independent twin-arginine translocation system can transport both tightly folded and malfolded proteins across the thylakoid membrane. J Biol Chem. (1998), 25; 273(52):34868-74 Non Patent Literature 3: Matthew G Hicks et al., Formation of functional Tat translocases from heterologous components. BMC Microbiology (2006), 6:64
Non Patent Literature 4: Anna M Albiniak et al., High-level secretion of a recombinant protein to the culture medium with a Bacillus subtilis twin-arginine translocation system in Escherichia coli. FEBS J. (2013), 280(16):3810-21.

### Summary of Invention

However, the microorganisms used in Patent Literatures 1 and 2 and Non Patent Literature 3 and 4 (Bacillus subtilis, coryneform bacteria, and E.coli) are microorganisms that all have an endogenous Tat system secretion apparatus (have endogenous genes that encode Tat proteins constituting the Tat system secretion apparatus), and are not focused on expressing heterologous proteins using microorganisms without the endogenous Tat system secretion apparatus (Protein J. 2019; 38(4): 377-388., Proc Natl Acad Sci USA. 2006 Nov 21;103(47):17927-32., Crit Rev Biotechnol. 2017 Jun;37(4):541-551.).

As a result of diligent research, the present inventors have found that even microorganisms without an endogenous Tat system secretion apparatus can secrete a heterologous protein using a heterologous Tat protein. This allows for the secretion of a greater quantity of protein even in microorganisms without an endogenous Tat system secretion apparatus. Specifically, the present invention includes the following aspects.
[1] A bacterium selected from the group consisting of the genus Bifidobacterium, the genus Lactococcus, the genus Lactobacillus, the genus Limosilactobacillus, and the genus Staphylococcus, which has been modified to express a heterologous Tat protein, with an ability to produce and secrete a heterologous protein.
[2] The bacterium according to [1], wherein the bacterium is a bacterial strain that does not have an endogenous Tat system secretion apparatus.
[3] The bacterium according to [1] or [2], which has an ability to extracellularly secrete the heterologous protein through a heterologous Tat protein.
[4] The bacterium according to any one of [1] to [3], wherein the bacterium is selected from the group consisting of Lactococcus Lactis, Bifidobacterium longum, Lactobacillus reuteri (also called Limosilactobacillus reuteri), and Staphylococcus epidermidis.
[5] The bacterium according to any one of [1] to [4], containing a genetic construct that includes, in a 5' to 3' direction, a nucleic acid sequence encoding a Tat system-dependent signal peptide and a nucleic acid sequence encoding a heterologous protein.
[6] The bacterium according to [5], wherein the genetic construct further includes a nucleic acid sequence encoding a heterologous Tat protein.
[7] The bacterium according to any one of [1] to [6], wherein the heterologous Tat protein is a Tat protein derived from bacteria selected from the genus Corynebacterium, the genus Bacillus, and the genus Bifidobacterium.
[8] The bacterium according to any one of [1] to [7], wherein the heterologous Tat protein includes at least one selected from TatA, TatB, and TatC.
[9] The bacterium according to any one of [1] to [8], wherein the heterologous Tat protein includes TatA, TatB and TatC.
[10] The bacterium according to any one of [1] to [9], wherein the heterologous protein includes at least one selected from FGF2 and FGF1.
[11] The bacterium according to any one of [5] to [10], wherein the genetic construct further includes a promoter derived from bacteria selected from the group consisting of the genus Bifidobacterium, the genus Lactococcus, the genus Lactobacillus, the genus Limosilactobacillus, and the genus Staphylococcus.
[12] The bacterium according to any one of [5] to [11], wherein the genetic construct further includes a nucleic acid sequence encoding a signal peptidase.
[13] The bacterium according to any one of [5] to [12], further containing a recombinant vector into which the genetic construct has been incorporated.
[14] A method for producing a heterologous protein, including: culturing the bacterium according to any one of [1] to [13] to produce and secrete a heterologous protein.
[15] A pharmaceutical composition containing the bacterium according to any one of [1] to [13].
[16] The pharmaceutical composition according to [15] for treating or preventing a disease or condition selected from cancer, lower limb ischemic diseases, rashes or intraoral mucositis accompanying cancer treatment, ichthyosis, cavities, irritable bowel syndrome, obesity, injuries, and surgical scars.

According to the present invention, even microorganisms without an endogenous Tat system secretion apparatus can express a heterologous protein using a heterologous Tat protein. This has the advantage that even proteins that are not secreted by the endogenous Sec system of the microorganisms can be produced and secreted, thereby expanding the range of utilization of the microorganisms.

### Brief Description of Drawings

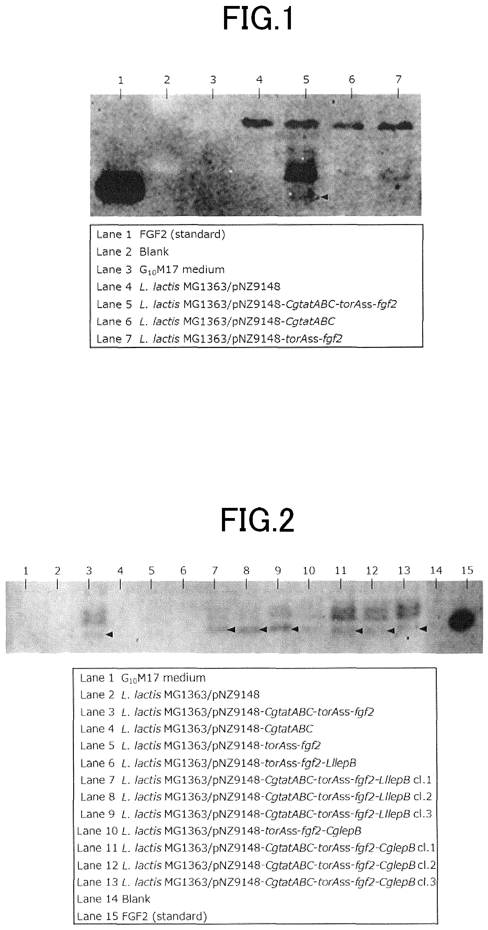

Fig. 1 shows whether the heterologous protein FGF2 was produced and secreted after culturing Lactococcus lactis/pNZ9148 strain (lane 4), Lactococcus lactis/pNZ9148-CgtatABC-torAss-fgf2 strain (lane 5), Lactococcus lactis/pNZ9148-CgtatABC strain (lane 6), and Lactococcus lactis/pNZ9148-torAss-fgf2 strain (lane 7). Among lanes 4 to 7, the production and secretion of FGF2 were confirmed only in lane 5.
Fig. 2 shows whether the TorA signal peptide attached to the heterologous protein FGF2 was cleaved, and the mature form of FGF2 was produced and secreted after culturing Lactococcus lactis/pNZ9148-CgtatABC-torAss-fgf2 strain (lane 3), Lactococcus lactis/pNZ9148-CgtatABC-torAss-fgfl-LllepB strain (lanes 7, 8, and 9), and Lactococcus lactis/pNZ9148-CgtatABC-torAss-fgf2-CglepB strain (lanes 10, 11, and 12). An increase in the production and secretion amount of the cleaved form of FGF2 was confirmed in lanes 7 to 9, and lanes 10, 11, and 12.

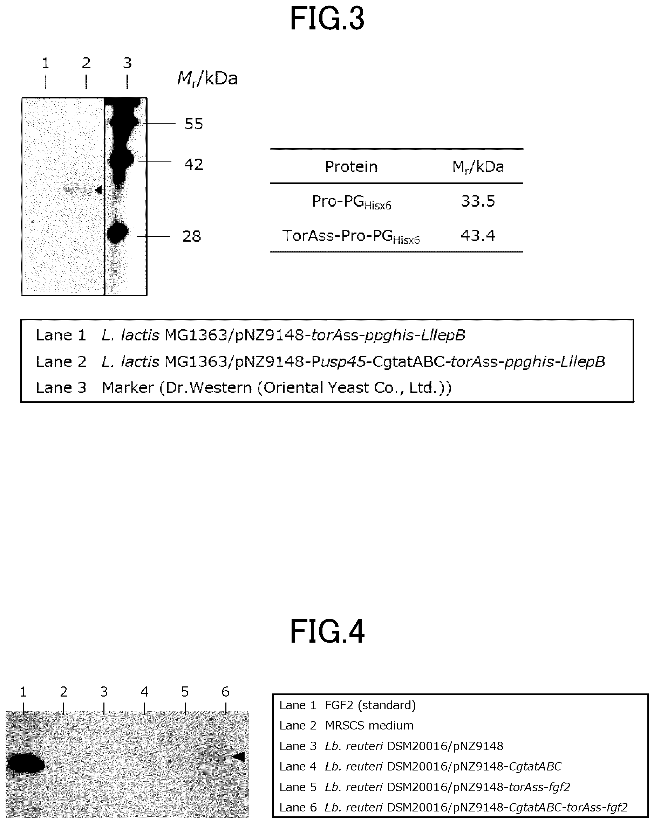

Fig. 3 shows whether the heterologous protein Pro-PGHisx6 was produced and secreted after culturing Lactococcus lactis/pNZ9148-torAss-ppghis-LllepB strain (lane 1) and Lactococcus lactis/pNZ9148-Pusp45-CgtatABC-torAss-ppghis-LllepB strain (lane 2). Among lanes 1 and 2, the production and secretion of Pro-PGHisx6 were confirmed only in lane 2.
Fig. 4 shows whether the heterologous protein FGF2 was produced and secreted after culturing Lactobacillus reuteri/pNZ9148 strain (lane 3), Lactobacillus reuteri/pNZ9148-CgtatABC strain (lane 4), Lactobacillus reuteri/pNZ9148-torAss-fgf2 strain (lane 5), and Lactobacillus reuteri/pNZ9148-CgtatABC-torAss-fgf2 strain (lane 6). Among lanes 3 to 6, the production and secretion of FGF2 were confirmed only in lane 6.

### Description of Embodiments

### <<Bacteria>>

The first embodiment of the present invention is a bacterium selected from the group consisting of the genus Bifidobacterium, the genus Lactococcus, the genus Lactobacillus, the genus Limosilactobacillus, and the genus Staphylococcus, which has been modified to express a heterologous Tat protein, with an ability to produce and secrete a heterologous protein.

Hereinafter, each configuration will be described.

In the present specification and in the scope of the patent claims, the "endogenous" proteins or genes of a microorganism refer to proteins or genes derived from that microorganism, and are used to distinguish from proteins or genes derived from "heterologous" organisms not derived from the microorganism. For example, the endogenous Tat system secretion apparatus or endogenous Tat protein of a microorganism refers to the Tat system secretion apparatus or Tat protein naturally possessed by that microorganism, and the endogenous Tat system secretion apparatus or endogenous Tat protein does not include the Tat system secretion apparatus or Tat protein when genes encoding the Tat system secretion apparatus or Tat protein derived from other organisms are incorporated and expressed in the microorganism.

The bacterium used in the present invention is a bacterium selected from the group consisting of the genus Bifidobacterium, the genus Lactococcus, the genus Lactobacillus, the genus Limosilactobacillus, and the genus Staphylococcus.

Bacteria of the genus Bifidobacterium include Bifidobacterium longum, Bifidobacterium breve, and Bifidobacterium bifidum. Preferable examples of bacteria of the genus Bifidobacterium include bacterial strains such as Bifidobacterium longum 105-A strain, Bifidobacterium longum JCM 1217 (ATCC 15707(Z. sun, et al., Front. Microbiol., 10, 796 (2009))), Bifidobacterium breve UCC2003 (A. N. Shkoporov, et al., FEMS Microbiol. Lett., 362, 12 (2015)), and Bifidobacterium bifidum S17 (Z. sun, et al., Bioengineered, 5:6, 371-377 (2014)).

Bacteria of the genus Lactococcus include Lactococcus lactis and Lactococcus raffinolactis. Among these, Lactococcus lactis is preferable, and the Lactococcus lactis subsp. cremoris is more preferable. Preferable examples of bacteria of the genus Lactococcus include bacterial strains such as Lactococcus lactis subsp. cremoris MG1363 strain, Lactococcus lactis subsp. cremoris SK11, and Lactococcus lactis subsp. lactis IL1403 (see US008759088B2, N. Noreen, et al., Microb. Cell. Fact., 10, 28 (2011), etc.).

Bacteria of the genus Lactobacillus and/or genus Limosilactobacillus include Lactobacillus plantarum, and Lactobacillus reuteri (also called Limosilactobacillus reuteri). Among these, Lactobacillus reuteri is preferable. Preferable examples of bacteria of the genus Lactobacillus and/or genus Limosilactobacillus include bacterial strains such as Lactobacillus reuteri DSM20016 strain, R2LC strain, ATCC PTA 6475 strain, and ATCC PTA 5290 (see EP3735979A1, EP1868622A1, EP1765975A1, etc.).

Note that Lactobacillus reuteri has been reclassified as Limosilactobacillus reuteri in recent years following a genome-level re-evaluation of the genus classification (Int J Syst Evol Microbiol. 2020 Apr; 70(4): 2782-2858), but it is still referred to by the name Lactobacillus reuteri in many papers.

In the present specification and in the claims, Lactobacillus reuteri is used interchangeably with Limosilactobacillus reuteri. Also, in the present specification and in the scope of the patent claims, bacteria of the genus Lactobacillus refer to not only the bacteria classified in the genus Lactobacillus at the time of filing of the present application but also generally refer to bacteria classified in the genus Lactobacillus at least at some point before the filing, at the time of the filing, and after the filing of the present application. If further limitation is necessary, the bacteria of the genus Lactobacillus may be the bacteria classified in the genus Lactobacillus at the time of filing of the present application.

Bacteria of the genus Staphylococcus include Staphylococcus epidermidis, Staphylococcus aureus, and Staphylococcus saprophyticus. Preferable examples of bacteria of the genus Staphylococcus include bacterial strains such as Staphylococcus epidermidis ATCC 12228 strain, Staphylococcus epidermidis NRRL B-4268 strain, and Staphylococcus epidermidis ATCC 14990 strain (see US20210162030, etc.).

The bacteria of the present invention are preferably bacterial strains that do not have an endogenous Tat system secretion apparatus (or do not have endogenous Tat proteins). That is, the bacteria of the present invention are preferably bacterial strains that do not have endogenous genes encoding Tat proteins constituting the Tat system secretion apparatus. Known bacterial strains that do not have an endogenous Tat system secretion apparatus include Bifidobacterium longum (especially strain 105-A), Lactococcus lactis, Staphylococcus epidermidis, Bifidobacterium breve (especially strains S-17, UCC2003, and LMG 13208), Bifidobacterium breve (especially strain S-27), Lactobacillus plantarum (especially WCSF1), and Lactobacillus reuteri (especially strain DSM20016) (Appl Environ Microbiol. 2006 Dec;72(12):7626-33., Appl Environ Microbiol. 2018 Aug 1;84(16):e00796-18., J Bacteriol. 2009 Oct; 191(19): 5921-5929., Proc Natl Acad Sci USA. 2003 Feb 18; 100(4): 1990-1995., Appl Environ Microbiol. 2003 Dec;69(12):6994-7001, PLoS One. 2015 Jun; 10(6): e0128802, FEMS Microbiol Rev. 2010 Mar;34(2):199-230.).

The bacteria used in the present invention are preferably bacteria selected from the group consisting of Lactococcus lactis, Bifidobacterium longum, Staphylococcus epidermidis, Bifidobacterium breve, Bifidobacterium bifidum, Lactobacillus plantarum, and Lactobacillus reuteri, more preferably bacteria selected from the group consisting of Lactococcus lactis, Bifidobacterium longum, Lactobacillus reuteri, and Staphylococcus epidermidis, and further preferably Lactococcus lactis.

Hereinafter, the bacteria or bacterial strains used in the present invention may be referred to as "hosts."

### <Ability to Produce and Secrete Heterologous Proteins>

The bacteria used in the present invention have the ability to produce and secrete a heterologous protein. Moreover, the bacteria used in the present invention preferably have the ability to extracellularly secrete a heterologous protein through heterologous Tat proteins. The ability to produce and secrete a heterologous protein can be acquired, for example, by the bacteria used in the present invention having a genetic construct for the production and secretion of a heterologous protein.

In the present invention, the term "protein" is a concept that includes not only proteins but also oligopeptides and polypeptides.

In the present specification, the "secretion" of a protein or peptide refers to the transfer of the protein or peptide molecules outside the bacterial cell body (extracellularly), encompassing not only cases where eventually the protein or peptide molecules are completely released into the medium but also cases where only some of them are present extracellularly or on the surface of the bacterial body.

In the present invention, having the "ability to produce and secrete" a heterologous protein means that when the bacteria of the present invention are cultured in a medium, the bacteria can produce a heterologous protein and secrete it outside the bacterial cell body.

### <Heterologous Protein>

In the present invention, "heterologous protein" refers to a foreign protein for the bacteria used in the present invention. The heterologous protein is preferably a protein derived from an organism other than bacteria selected from the group consisting of the genus Bifidobacterium, the genus Lactococcus, the genus Lactobacillus, the genus Limosilactobacillus, and the genus Staphylococcus. The heterologous protein may be a protein derived from animals, a protein derived from plants, a protein derived from bacteria, a protein derived from fungi, or a protein derived from viruses. Among these, the heterologous protein is preferably a protein derived from animals or a protein derived from bacteria, further more preferably a protein derived from animals, and particularly more preferably a protein derived from mammals. In addition, a protein derived from mammals is preferably a protein derived from humans.

Also, the heterologous protein may be a protein having the same amino acid sequence as the protein endogenous in organisms other than bacteria selected from the group consisting of the genus Bifidobacterium, the genus Lactococcus, the genus Lactobacillus, the genus Limosilactobacillus, and the genus Staphylococcus, or it may be a protein that has modified the amino acid sequence of the protein endogenous in the aforementioned organisms. Examples of proteins that have modified the amino acid sequence of proteins endogenous in the aforementioned organisms include proteins with improved enzymatic activity.

There are no particular limitations on the heterologous protein produced and secreted by the bacteria of the present invention, and any desired heterologous protein may be used. Specific examples of a heterologous protein include human-derived fibroblast growth factors (FGF) such as human-derived basic fibroblast growth factor (bFGF or FGF2) and human-derived acidic fibroblast growth factor (aFGF or FGF1), protein-glutaminase, isomaltodextranase, and trans-glutaminase. Among these, it is preferable to include FGF, preferable to include at least one selected from FGF2 and FGF1, more preferable to be at least one selected from FGF2 and FGF1, and further preferable to be FGF2.

The heterologous protein may have a pre-sequence (signal sequence) and/or pro-sequence that can be possessed in its natural state, or it may be such that such pre-sequence and/or pro-sequence has been removed. Also, the heterologous protein may be one to which a sequence not originally possessed, for example, a desired signal sequence or a functional sequence such as a tag sequence, has been added.

### <Tat System-Dependent Signal Peptide (Signal Sequence)>

In the present invention, the Tat system-dependent signal peptide refers to a signal peptide recognized by the Tat system. The Tat system-dependent signal peptide possesses an arginine-arginine sequence, and by the Tat proteins (or Tat secretion apparatus) recognizing this sequence, it can secrete proteins having this signal peptide at the N-terminus to outside the bacterial body (extracellular).

A heterologous protein may or may not be linked to the Tat system-dependent signal peptide at their N-terminus. By the heterologous protein having the Tat system-dependent signal peptide at their N-terminus, the Tat system can recognize the arginine-arginine sequence in the signal peptide and can secrete the heterologous protein from inside the bacterial body (intracellular) to outside the bacterial body (extracellular), but the Tat system-dependent signal peptide can be cleaved from the heterologous protein either simultaneously with secretion or thereafter. Therefore, the heterologous protein being produced and secreted may or may not be linked to the Tat system-dependent signal peptide. Preferably, the heterologous protein being produced and secreted are not linked to the Tat system-dependent signal peptide.

Examples of the Tat system-dependent signal peptide include the signal peptide of Escherichia coli trimethylamine N-oxide reductase (TorA) (SEQ ID NO: 49), the signal peptide of Escherichia coli Sufl (suppressor of ftsl; ftsl suppressor) (SEQ ID NO: 50), the signal peptide of Bacillus subtilis-derived PhoD (phosphodiesterase) (SEQ ID NO: 51), the signal peptide of Streptomyces lividans-derived XlnC (xylanase) (SEQ ID NO: 52), the signal peptide of Arthrobacter globiformis-derived isomaltodextranase (IMD) (SEQ ID NO: 53), and other signal peptides, including the signal peptide of Bifidobacterium longum-derived FAD-dependent oxidoreductase (SEQ ID NO: 72).

Among these, preferably as the Tat system-dependent signal peptides are the signal peptide of trimethylamine N-oxide reductase derived from Escherichia coli (TorA signal peptide, TorAss) and the signal peptide of FAD-dependent oxidoreductase derived from Bifidobacterium longum, with the signal peptide of trimethylamine N-oxide reductase derived from Escherichia coli (TorA signal peptide, TorAss) being more preferable.

Examples of the amino acid sequences of the signal peptides include amino acid sequences having 70% or more (preferably 80% or more, more preferably 90% or more, further more preferably 95% or more, further more preferably 98% or more, further more preferably 99% or more, and most preferably 100%) sequence identity with any of the amino acid sequences of SEQ ID NOs: 49 to 53 and 72 (preferably, the amino acid sequence of SEQ ID NO: 49 being the amino acid sequence of the signal peptide of trimethylamine N-oxide reductase derived from Escherichia coli, or the amino acid sequence of SEQ ID NO: 72 being the amino acid sequence of the signal peptide of FAD-dependent oxidoreductase derived from Bifidobacterium longum).

As stated above, a heterologous protein may or may not have a part or all of the signal peptide removed by the endogenous or heterologous signal peptidases possessed by the host when secreted extracellularly through the Tat system secretion apparatus (Tat system, Tat pathway). Therefore, the heterologous protein produced and secreted by the bacteria of the present invention may be a heterologous protein with the signal peptide completely removed, that is, not retaining any signal peptide (fully cleaved type of a heterologous protein), a heterologous protein with a part of the signal peptide removed (partially cleaved type of a heterologous protein), or a heterologous protein having the signal peptide (uncleaved type of a heterologous protein). Preferably, it is a heterologous protein with the signal peptide completely removed.

### <Tat Protein>

In the present invention, the "Tat system" refers to the pathway also known as the "Twin-arginine translocation pathway," a mechanism or pathway that recognizes a conserved region of arginine-arginine retained in the signal peptide to secrete proteins. Proteins in the Tat system can include TatA, TatB, and TatC. In the present invention, the "Tat system secretion apparatus" refers to membrane proteins constituted by one or more of TatA, TatB, and TatC. TatA, TatB, and TatC are transmembrane proteins existing in the cell membrane, and they are believed to form pores for the translocation of proteins on the cell membrane.

In the present invention, "Tat proteins" refer to membrane-permeable proteins used in the Tat system. Tat proteins can include TatA, TatB, and TatC.

TatA is the most abundant component of the Tat complex and is believed to be most involved in the formation of protein transport channels. TatB is known to bind with the Tat system-dependent signal peptide and later play a role in binding with the mature protein. TatC is known to play a role of assisting in binding with the proteins to be transported (Protein J. 2019; 38(4): 377-388).

The Tat system secretion apparatus exists in a configuration constituted by three kinds of proteins, TatA, TatB, and TatC, or two kinds of proteins, TatA and TatC, depending on the biological species, and in the latter case, it is known that TatA also has the function of TatB. Note that in the latter case, for example, in Bacillus subtilis, proteins equivalent to TatA are known as TatAd and TatAy, and proteins equivalent to TatC are known as TatCd and TatCy. In the present invention, "TatA" is a concept encompassing not only the TatA of various microorganisms but also TatAd and TatAy of Bacillus subtilis. Similarly, "TatC" is a concept encompassing not only the TatC of various microorganisms but also TatCd and TatCy of Bacillus subtilis.

The heterologous Tat proteins expressed by the bacteria of the present invention preferably include at least one selected from TatA, TatB, and TatC, more preferably include TatA and TatC, and most preferably include TatA, TatB, and TatC.

In the present invention, "heterologous Tat protein" refers to a Tat protein that is foreign to the bacteria used in the present invention. The heterologous protein may be a plant-derived Tat protein, or may be a bacterium-derived Tat protein, but it is preferably a bacterium-derived Tat protein, more preferably a Tat protein derived from bacteria selected from the genus Corynebacterium, the genus Bacillus, and the genus Bifidobacterium, and further more preferably a Tat protein derived from bacteria of the genus Corynebacterium or the genus Bacillus.

Examples of bacteria of the genus Corynebacterium include Corynebacterium glutamicum, Corynebacterium ammoniagenes, and Corynebacterium casei. Among these, Corynebacterium glutamicum is preferable, and the C. glutamicum 2256 strain is more preferable.

Bacteria of the genus Bacillus include Bacillus subtilis and Bacillus thuringiensis. Among these, Bacillus subtilis is preferable, and the Bacillus subtilis 168 strain is more preferable.

Among these, it is preferable to be a Tat protein derived from bacteria of the genus Corynebacterium, and it is more preferable to be a Tat protein derived from Corynebacterium glutamicum.

The heterologous Tat protein may be a Tat protein having the same amino acid sequence as the natural Tat protein endogenous in the aforementioned organisms, or it may be a modified Tat protein that has altered the amino acid sequence to the extent that it does not impair the function of the Tat protein endogenous in the aforementioned organisms, but it is preferable to be a Tat protein having the same amino acid sequence as the natural Tat protein endogenous in the aforementioned organisms.

As for TatA, TatB, and TatC proteins, they may be TatA, TatB, and TatC proteins derived from the aforementioned organisms. Among these, it is preferable to be TatA, TatB, and TatC proteins derived from Corynebacterium glutamicum, or TatAd, TatCd, TatAy, and TatCy proteins derived from Bacillus subtilis, or TatA, TatB, and TatC proteins derived from Bifidobacterium longum (preferably from Bifidobacterium longum E-18 strain).

The TatA protein, TatB protein, and TatC protein derived from Corynebacterium glutamicum have amino acid sequences of SEQ ID NOs: 24, 25, and 26, respectively.

The TatAd protein, TatCd protein, TatAy protein, and TatCy protein derived from Bacillus subtilis have amino acid sequences of SEQ ID NOs: 39, 40, 41, and 42, respectively.

The TatA protein, TatB protein, and TatC protein derived from Bifidobacterium longum E-18 strain have amino acid sequences of SEQ ID NOs: 46, 47, and 48, respectively.

As to the heterologous TatA used in the present invention, there are no particular limitations on the amino acid sequence thereof as long as it retains the membrane penetration function possessed by TatA having the amino acid sequence of SEQ ID NO: 24, 39, 41, or 46, but it is preferable to have an amino acid sequence having 70% or more sequence identity with the amino acid sequence of SEQ ID NO: 24, 39, 41, or 46, more preferably have an amino acid sequence having 80% or more sequence identity, further more preferably have an amino acid sequence having 90% or more sequence identity, further more preferably have an amino acid sequence having 95% or more sequence identity, further more preferably have an amino acid sequence having 98% or more sequence identity, further more preferably have an amino acid sequence having 99% or more sequence identity, and further more preferably have an amino acid sequence having 100% sequence identity.

As to the heterologous TatB used in the present invention, there are no particular limitations on the amino acid sequence thereof as long as it retains the membrane penetration function possessed by TatB having the amino acid sequence of SEQ ID NO: 25 or 47, but it is preferable to have an amino acid sequence having 70% or more sequence identity with the amino acid sequence of SEQ ID NO: 25 or 47, more preferably have an amino acid sequence having 80% or more sequence identity, further more preferably have an amino acid sequence having 90% or more sequence identity, further more preferably have an amino acid sequence having 95% or more sequence identity, further more preferably have an amino acid sequence having 98% or more sequence identity, further more preferably have an amino acid sequence having 99% or more sequence identity, and further more preferably have an amino acid sequence having 100% sequence identity.

As to the heterologous TatC used in the present invention, there are no particular limitations on the amino acid sequence thereof as long as it retains the membrane penetration function possessed by TatC having the amino acid sequence of SEQ ID NO: 26, 40, 42, or 48, but it is preferable to have an amino acid sequence having 70% or more sequence identity with the amino acid sequence of SEQ ID NO: 26, 40, 42, or 48, more preferably have an amino acid sequence having 80% or more sequence identity, further more preferably have an amino acid sequence having 90% or more sequence identity, further more preferably have an amino acid sequence having 95% or more sequence identity, further more preferably have an amino acid sequence having 98% or more sequence identity, further more preferably have an amino acid sequence having 99% or more sequence identity, and further more preferably have an amino acid sequence having 100% sequence identity.

### <Signal Peptidase>

In the present invention, "signal peptidase" refers to a protein (enzyme) that has the activity to remove part or all of the signal peptide from proteins possessing a Tat system-dependent signal peptide.

The bacteria of the present invention preferably express a signal peptidase that removes part or all (preferably all) of the Tat system-dependent signal peptide from proteins possessing the Tat system-dependent signal peptide.

As for the signal peptidase, there are no particular limitations as long as it possesses the aforementioned activity, but it is preferable to have four conserved regions, BoxB, BoxC, BoxD, and BoxE, in the amino acid sequence (Pharmacol. Ther. 2000 87: 27-49), more preferable to be type I signal peptidase derived from Escherichia coli (EcLepB; Mol. Gen. Genet. 1991 227; 40-44.), type I signal peptidase derived from Bacillus subtilis (SipS, SipT; Genes Dev. 1998 12; 2318-2331.), type I signal peptidase derived from Corynebacterium glutamicum (C. glutamicum) strain 2256 (CgLepB; WO 2020/085511), type I signal peptidase derived from Lactococcus lactis (L. lactis) strain MG1363 (LlLepB), type I signal peptidase derived from Lactobacillus reuteri (L. reuteri) (LrLepB), type I signal peptidase derived from Bifidobacterium longum (B. longum) (BlLepB), and type I signal peptidase derived from Staphylococcus (S. epidermidis) (SeLepB), with Corynebacterium glutamicum (C. glutamicum) strain 2256-derived type I signal peptidase (CgLepB; WO 2020/085511) and Lactococcus lactis (L. lactis) strain MG1363-derived type I signal peptidase (LlLepB) being particularly preferable.

The amino acid sequence of each of the aforementioned signal peptidases is as follows.
Amino acid sequence of EcLepB: SEQ ID NO: 30
Amino acid sequence of SipS: SEQ ID NO: 31
Amino acid sequence of SipT: SEQ ID NO: 32
Amino acid sequence of CgLepB: SEQ ID NO: 33
Amino acid sequence of LlLepB: SEQ ID NO: 34
Amino acid sequence of LrLepB: SEQ ID NO: 74
Amino acid sequence of BlLepB: SEQ ID NO: 75
Amino acid sequence of SeLepB: SEQ ID NO: 78

The amino acid sequence of the signal peptidase is preferably an amino acid sequence having 70% or more (preferably 80% or more, more preferably 90% or more, further more preferably 95% or more, further more preferably 98% or more, further more preferably 99% or more, and most preferably 100%) sequence identity with the amino acid sequence of SEQ ID NO: 30, 31, 32, 33, 34, 74, 75, or 78.

### <Other Configurations>

The bacterium of the present invention may have an endogenous protease gene that is deficient or has reduced expression. The deficiency or reduction in the expression of the protease gene can prevent the degradation of the protein to be produced and secreted.

### <Genetic Construct>

In the present invention, a "genetic construct" refers to something that possesses, at appropriate positions where they can function, a promoter, sequences encoding appropriate signal peptides and a nucleic acid fragment encoding the target protein, and control sequences necessary to express the target protein gene (such as operators and terminators).

The bacterium of the present invention preferably contains a genetic construct including a nucleic acid sequence encoding a heterologous protein. Also, it is preferable that the genetic construct includes, in the 5' to 3' direction, a nucleic acid sequence encoding a Tat system-dependent signal peptide and a nucleic acid sequence encoding a heterologous protein. At this time, there is no stop codon between the nucleic acid sequence encoding the Tat system-dependent signal peptide and the nucleic acid sequence encoding the heterologous protein. This allows for the production in the host bacterium of the heterologous protein to which the Tat system-dependent signal peptide is linked at the N-terminus.

Furthermore, the genetic construct preferably includes a nucleic acid sequence encoding a heterologous Tat protein. The nucleic acid sequence encoding the heterologous Tat protein may be located at the 5'-terminus side of the nucleic acid sequence encoding the Tat system-dependent signal peptide, or at the 3'-terminus side of the nucleic acid sequence encoding the heterologous protein. Also, the nucleic acid sequence encoding the heterologous Tat protein and the nucleic acid sequence encoding the heterologous protein may exist within the same genetic construct, or they may each exist within different genetic constructs.

### <Vector>

The bacteria used in the present invention preferably contain a recombinant vector in which the genetic construct has been incorporated. Examples of vectors used to incorporate the genetic construct include, but are not limited to, plasmids, transposons, and phages. As for the plasmids, there are no particular limitations as long as it is possible to incorporate the genetic construct in an appropriate arrangement, and various plasmids can be used. Concrete examples of plasmids that can be used in the present invention include the pNZ8148 vector (manufactured by GoldBio, SEQ ID NO: 62), pBS423 (Appl. Environ. Microbiol. 2012 Jun; 78(14): 4984-4944, SEQ ID NO:63), pRMC2 (Plasmid. 2009 Mar; 61(2): 126-9.), and the like.

There are no particular limitations on the method for incorporating the genetic construct or each gene, promoter sequences, or the like contained in the genetic construct into the vector, and for example, it can be incorporated into the vector by connecting the genetic construct to the cleavage site obtained by digesting a specific sequence of the vector with a restriction enzyme.

### <Nucleic Acid Sequence Encoding Heterologous Tat Protein>

The genetic construct or vector preferably includes a nucleic acid sequence encoding a heterologous Tat protein.

In the present invention, "nucleic acid sequence encoding a heterologous Tat protein" refers to a nucleic acid sequence encoding a Tat protein that is foreign to the bacteria used in the present invention. The aforementioned can be given as examples of heterologous Tat proteins.

The nucleic acid sequence encoding a heterologous Tat protein preferably includes nucleic acid sequences encoding at least one Tat protein selected from TatA, TatB, and TatC, more preferably includes nucleic acid sequences encoding TatA (tatA) and nucleic acid sequences encoding TatC (tatC), and further more preferably includes nucleic acid sequences encoding TatA (tatA), nucleic acid sequences encoding TatB (tatB), and nucleic acid sequences encoding TatC (tatC).

As nucleic acid sequences encoding TatA, TatB, and TatC proteins, they may be nucleic acid sequences encoding the aforementioned biologically derived TatA, TatB, and TatC proteins. Among these, it is preferable to be a nucleic acid sequence encoding TatA, TatB, and TatC proteins derived from Corynebacterium glutamicum, or TatAd, TatCd, TatAy, and TatCy proteins derived from Bacillus subtilis, or TatA, TatB, and TatC derived from Bifidobacterium longum (preferably from Bifidobacterium longum E-18 strain).

The nucleic acid sequences encoding TatA protein, TatB protein, and TatC protein derived from Corynebacterium glutamicum have nucleic acid sequences of SEQ ID NOs: 4, 5, and 6, respectively.

The nucleic acid sequences encoding TatAd protein, TatCd protein, TatAy protein, and TatCy protein derived from Bacillus subtilis have nucleic acid sequences of SEQ ID NOs: 35, 36, 37, and 38, respectively.

The nucleic acid sequences encoding TatA, TatB, and TatC proteins derived from Bifidobacterium longum E-18 have nucleic acid sequences of SEQ ID NOs: 43, 44, and 45, respectively.

The nucleic acid sequences encoding the TatA, TatB, and TatC proteins used in the present invention are preferably nucleic acid sequences having 70% or more (preferably 80% or more, more preferably 90% or more, further more preferably 95% or more, further more preferably 98% or more, further more preferably 99% or more, and most preferably 100%) sequence identity with the above nucleic acid sequences.

Furthermore, the nucleic acid sequences encoding heterologous TatA, TatB, and TatC may be optimized according to the bacterium to serve as a host (note that it is preferable keep the amino acid sequences of TatA, TatB, and TatC unchanged and optimize the nucleic acid sequences encoding them). For example, it is possible to enhance the translation efficiency of a gene by substituting rare codons present in the gene with synonymous codons used more frequently. That is, the gene to be introduced may be modified to have optimal codons according to the codon usage frequency of the host to be used. Codon substitution can be carried out, for example, by a site-specific mutation method that introduces the target mutation into the target portion of DNA. Also, a gene fragment with substituted codons may be entirely synthesized. The codon usage frequencies in various organisms are disclosed in "Codon Usage Database" (http://www.kazusa.or.jp/codon; Nakamura, Y. et al, Nucl. Acids Res., 28, 292 (2000)).

Nucleic acid sequences preferably have 70% or more (preferably 80% or more, more preferably 90% or more, further more preferably 95% or more, further more preferably 98% or more, further more preferably 99% or more, and most preferably 100%) sequence identity with SEQ ID NOs: 15, 16, and 17 in the case of Lactococcus lactis and Lactobacillus reuteri, SEQ ID NOs: 18, 19, 20 in the case of Bifidobacterium longum, and SEQ ID NOs: 21, 22, 23 in the case of Staphylococcus epidermidis, which are optimized from TatA, TatB, and TatC of Corynebacterium glutamicum.

Moreover, the nucleic acid sequences encoding the heterologous TatA, TatB, and TatC used in the present invention preferably have nucleic acid sequences corresponding to the amino acid sequences described in the above section "Heterologous Tat Proteins".

In a genetic construct or vector, the nucleic acid sequence encoding the heterologous Tat proteins may be located on the 5'-side of the nucleic acid sequence encoding the Tat system-dependent signal peptide described later, or on the 3'-side of the nucleic acid sequence encoding the heterologous protein, but it is preferable to be located on the 5'-side.

Furthermore, in a genetic construct or vector, the nucleic acid sequence encoding heterologous Tat proteins is preferably located on the 3'-side of the promoter sequence described later.

Most preferably, in a genetic construct or vector, they are arranged in the order from 5' to 3' direction as the promoter sequence, the nucleic acid sequence encoding heterologous Tat proteins, the nucleic acid sequence encoding the Tat system-dependent signal peptide, and the nucleic acid sequence encoding the heterologous protein.

### <Nucleic Acid Sequence Encoding Tat System-Dependent Signal Peptide>

The genetic construct or vector preferably further includes a nucleic acid sequence encoding the Tat system-dependent signal peptide. It is preferable that the nucleic acid sequence encoding the Tat system-dependent signal peptide has, on the 3'-terminus side thereof, a nucleic acid sequence encoding a heterologous protein. Specifically, it is preferable that the genetic construct includes, from the 5' to 3' direction, a nucleic acid sequence encoding the Tat system-dependent signal peptide and a nucleic acid sequence encoding a heterologous protein. Additionally, the nucleic acid sequence encoding the Tat system-dependent signal peptide and the nucleic acid sequence encoding a heterologous protein are preferably configured such that, when expressed, the Tat system-dependent signal peptide is directly linked to the N-terminus of the heterologous protein.

Examples of the Tat system-dependent signal peptides include those described in the above "Signal Peptide (Signal Sequence)" section. The nucleic acid sequence encoding a signal peptide can also be optimized according to the host. Optimization methods include, for example, those similar to the methods described in the "Nucleic Acid Sequence Encoding Heterologous Tat Protein" section.

The nucleic acid sequence encoding the Tat system-dependent signal peptide is preferably a nucleic acid sequence having 70% or more (preferably 80% or more, more preferably 90% or more, further more preferably 95% or more, further more preferably 98% or more, further more preferably 99% or more, and most preferably 100%) sequence identity with the nucleic acid sequence encoding the signal peptide of trimethylamine N-oxide reductase derived from Escherichia coli (SEQ ID NO: 10), or the nucleic acid sequence encoding the signal peptide of FAD-dependent oxidoreductase derived from Bifidobacterium longum (SEQ ID NO: 73).

Moreover, a nucleic acid sequence is also preferable that has 70% or more (preferably 80% or more, more preferably 90% or more, further more preferably 95% or more, further more preferably 98% or more, further more preferably 99% or more, and most preferably 100%) sequence identity with SEQ ID NO: 27, SEQ ID NO: 28, or SEQ ID NO: 29, which are obtained by optimizing the nucleic acid sequence encoding the signal peptide of trimethylamine N-oxide reductase derived from Escherichia coli for Lactococcus lactis and Lactobacillus reuteri, Bifidobacterium longum, or Staphylococcus epidermidis, respectively.

### <Nucleic Acid Sequence Encoding Signal Peptidase>

In the present invention, "signal peptidase" refers to a protein (enzyme) that has the activity to remove part or all of the signal peptide from proteins possessing a Tat system-dependent signal peptide.

Genetic constructs or vectors may further contain a nucleic acid sequence encoding signal peptidase. By including a nucleic acid sequence encoding signal peptidase, after the heterologous protein having the Tat system-dependent signal peptide on the N-terminal side thereof have been secreted extracellularly via the Tat system, it becomes easier to remove part or all of the Tat system-dependent signal peptide from the heterologous protein.

Examples of signal peptidases include those described in the "Signal Peptidase" section. Note that the nucleic acid sequences encoding the signal peptidases EclepB, SipS, SipT, CglepB, LllepB, LrlepB, and BllepB are as follows:
Nucleic acid sequence encoding EcLepB: SEQ ID NO: 80
Nucleic acid sequence encoding SipS: SEQ ID NO: 81
Nucleic acid sequence encoding SipT: SEQ ID NO: 82
Nucleic acid sequence encoding CgLepB: SEQ ID NO: 83
Nucleic acid sequence encoding LlLepB: SEQ ID NO: 84
Nucleic acid sequence encoding LrLepB: SEQ ID NO: 76
Nucleic acid sequence encoding BlLepB: SEQ ID NO: 77
Nucleic acid sequence encoding SeLepB: SEQ ID NO: 79

The nucleic acid sequence encoding signal peptidase can be optimized according to the host bacterium, or it may not be optimized. Methods for optimizing the nucleic acid sequence according to the host bacterium include, for example, those similar to the methods described in the "Nucleic Acid Sequence Encoding Heterologous Tat Protein" section.

The nucleic acid sequence encoding signal peptidase is preferably a nucleic acid sequence having 70% or more (preferably 80% or more, more preferably 90% or more, further more preferably 95% or more, further more preferably 98% or more, further more preferably 99% or more, and most preferably 100%) sequence identity with SEQ ID NO: 80, 81, 82, 83, 84, 76, 77, or 79.

Moreover, a nucleic acid sequence is also preferable that has 70% or more (preferably 80% or more, more preferably 90% or more, further more preferably 95% or more, further more preferably 98% or more, further more preferably 99% or more, and most preferably 100%) sequence identity with SEQ ID NO: 64, which is obtained by optimizing the nucleic acid sequence encoding signal peptidase I derived from Corynebacterium glutamicum for Lactococcus lactis and Lactobacillus reuteri.

### <Nucleic Acid Sequence Encoding Heterologous Protein>

A nucleic acid sequence encoding a heterologous protein is preferably a nucleic acid sequence encoding a protein derived from an organism other than bacteria selected from the group consisting of the genus Bifidobacterium, the genus Lactococcus, the genus Lactobacillus, the genus Limosilactobacillus, and the genus Staphylococcus. The aforementioned can be given as examples of a heterologous protein.

The nucleic acid sequence encoding a heterologous protein can be optimized according to the host bacterium, or it may not be optimized. Methods for optimizing the nucleic acid sequence according to the host bacterium include, for example, those similar to the methods described in the "Nucleic Acid Sequence Encoding Heterologous Tat Protein" section.

It is preferable that the nucleic acid sequence encoding a heterologous protein has, on the 5'-terminus side thereof, a nucleic acid sequence encoding the Tat system-dependent signal peptide. Specifically, it is preferable that the genetic construct includes, from the 5' to 3' direction, a nucleic acid sequence encoding the Tat system-dependent signal peptide and a nucleic acid sequence encoding a heterologous protein.

Additionally, the nucleic acid sequence encoding the Tat system-dependent signal peptide and the nucleic acid sequence encoding a heterologous protein are preferably configured such that there exists no stop codon therebetween, and the heterologous protein is expressed with the N-terminal thereof connected to the signal peptide.

### <Promoter>

A genetic construct or vector preferably further includes a promoter. The promoter is preferably positioned on the 5'-terminus side of the nucleic acid sequence encoding the Tat system-dependent signal peptide and the nucleic acid sequence encoding a heterologous protein.

The promoter is not particularly limited as long as it functions as a promoter within the host bacterium, but it is preferably a promoter derived from bacteria selected from the group consisting of the genus Bifidobacterium, the genus Lactococcus, the genus Lactobacillus, the genus Limosilactobacillus, and the genus Staphylococcus. Examples of such promoters include the pepN gene promoter (SEQ ID NO: 1) derived from Lactococcus lactis, the dnaJ gene promoter (SEQ ID NO: 55) derived from Lactococcus lactis, the tuf gene promoter (SEQ ID NO: 56) derived from Lactococcus lactis, the pfkA gene promoter (SEQ ID NO: 57) derived from Lactococcus lactis, the usp45 gene promoter (SEQ ID NO: 58) derived from Lactococcus lactis, the cbaH gene promoter (SEQ ID NO: 59) derived from Bifidobacterium longum, the gapA gene promoter (SEQ ID NO: 60) derived from Bifidobacterium longum, the tuf gene promoter (SEQ ID NO: 61) derived from Bifidobacterium longum, the Pxyl/tetO promoter (SEQ ID NO: 54) derived from Bacillus subtilis, and the tuf gene promoter (SEQ ID NO: 71) derived from Lactobacillus reuteri. Among these, the pepN gene promoter (PpepN), the cbaH gene promoter (PcbaH), Pxyl/tetO, and the like are preferable.

Additionally, the promoter can be a natural promoter derived from the aforementioned bacteria or a promoter obtained by modifying a natural promoter. Modifications to natural promoters include bringing the -35 and -10 regions within the promoter region closer to the consensus sequence (WO00/18935) and random modifications of non-consensus sequences (Microbiology 2006 Dec; 152: 1011-1019).

The promoter sequence has no particular limitations as long as it functions as a promoter within the host bacterium (has the function to transcriptionally control the nucleic acid sequence encoding a heterologous protein), but it is preferably a nucleic acid sequence having 70% or more (preferably 80% or more, even more preferably 90% or more, further more preferably 95% or more, further more preferably 98% or more, and most preferably 100%) sequence identity with the nucleic acid sequences of the aforementioned sequence numbers.

Also, the genetic construct or vector of the present invention preferably includes, in addition to the promoter sequence, a Shine-Dalgarno sequence (SD sequence). As for the Shine-Dalgarno sequence (SD sequence), it is preferable to include the nucleic acid sequence AGGAG.

### <Others>

Genetic constructs or vectors can be equipped with terminators for transcription termination. It is preferable that the terminator is arranged downstream of the nucleic acid sequence encoding a heterologous protein and/or a signal peptidase and/or a Tat protein. The terminator is not particularly limited as long as it functions in the bacterium according to the present invention. The terminator can be a host-derived terminator or it may be a heterologously derived terminator. The terminator may be a terminator inherent to the introduced gene or may be a terminator from another gene.

Vectors, promoters, and terminators that can be used in various microorganisms are described in detail, for example, in "Biseibutsugaku Kiso Kouza 8, Idenshi Kougaku, Kyoritsu Shuppan, 1987", and they can be utilized.

Furthermore, when introducing two or more genes, it suffices if each gene is retained in a bacterium of the present invention in a manner that allows expression. For example, all genes may be retained on a single expression vector or all may be located on a chromosome. Also, each gene can be separately retained on multiple expression vectors or separately on one or more expression vectors and on a chromosome. Moreover, two or more genes can be introduced in an operon format.

### <Introduction Method>

The method for introducing the genetic construct or recombinant vector, which can be used in the present invention, into the host bacterium is not particularly limited and can employ conventionally known methods. For example, as reported for Escherichia coli K-12, methods to increase the permeability of DNA by treating recipient bacterial cells with calcium chloride (Mandel, M. and Higa, A., J. Mol. Biol. 1970, 53, 159-162) or, as reported for Bacillus subtilis, methods to introduce DNA by preparing competent cells from growing cells (Duncan, C. H., Wilson, G. A. and Young, F. E., 1977. Gene 1: 153-167) can be used. Alternatively, as known for Bacillus subtilis, actinomycetes, and yeast, methods to introduce recombinant DNA into DNA-recipient bacteria by converting the cells of the DNA-recipient bacteria into a state of protoplast or spheroplast that easily takes up recombinant DNA (Chang, S. and Choen, S. N., 1979. Mol. Gen. Genet. 168: 111-115; Bibb, M. J., Ward, J. M. and Hopwood, O. A. 1978. Nature 274: 398-400; Hinnen, A., Hicks, J. B. and Fink, G. R. 1978. Proc. Natl. Acad. Sci. USA 75: 1929-1933) can also be applied. Alternatively, as reported for coryneform bacteria, the electric pulse method (Japanese Patent Application Publication No. Hei 2-207791) can also be utilized.

### <Production Method>

The second embodiment of the present invention is a method for producing a heterologous protein, including culturing the aforementioned bacterium to produce and secrete a heterologous protein.

There are no particular limitations on the method for culturing the bacterium, and known culture methods can be used.

The production method of the present invention may include culturing the bacterium to produce and secrete a heterologous protein, followed by removing the bacterial bodies. The removal methods include centrifugation, filtration, and the like.

Additionally, after collection, there may be a step to purify the heterologous protein. Purification methods include salting out, ethanol precipitation, ultrafiltration, gel filtration chromatography, ion-exchange column chromatography, affinity chromatography, medium- or high-pressure liquid chromatography, reverse-phase chromatography, and hydrophobic chromatography, among others.

Furthermore, during or after the culturing of the bacteria, there may be a step to add signal peptidase to the bacterial bodies or a culture solution containing the bacterial bodies.

### <Pharmaceutical Composition>

The third embodiment of the present invention is a pharmaceutical composition containing the aforementioned bacterium. In addition to bacteria, the pharmaceutical composition of the present invention may also contain a pharmaceutically acceptable carrier or diluent. Examples of carriers or diluents include water (such as water for injection and physiological saline), non-volatile oils, polyethylene glycol, glycerin, propylene glycol, antioxidants (such as ascorbic acid and sodium bisulfite), chelating agents (such as ethylenediaminetetraacetic acid), buffer agents (such as acetates, citrates, and phosphates), and isotonic agents (such as sodium chloride and glucose).

Dosage forms of the pharmaceutical composition include liquids, solid preparations, creams, gels, patches, and spray preparations (including inhalants).

For liquids, for example, the bacterium of the present invention can be placed in physiological saline or the like, and medicinal additives can be added as needed to produce a liquid. Medicinal additives include antioxidants (such as ascorbic acid and sodium bisulfite), chelating agents (such as ethylenediaminetetraacetic acid), buffer agents (such as acetates, citrates, and phosphates), isotonic agents (such as sodium chloride and glucose).

For solid preparations, for example, physiological saline containing the bacterium of the present invention or the aforementioned liquid can be freeze-dried to produce a freeze-dried preparation. Alternatively, physiological saline containing the bacterium of the present invention or the aforementioned liquid can be placed in a container and frozen to create a frozen preparation. Freeze-dried preparations and frozen preparations can also include medicinal additives as necessary. The medicinal additives include excipients, binders, disintegrators, lubricants, isotonic agents, stabilizers, and preservatives.

Furthermore, a mixture of the bacterium of the present invention with medicinal additives and the like can also be used as a solid preparation. In this case, it is preferable to refrigerate the mixture of the bacterium of the present invention with medicinal additives and the like until use. The medicinal additives include excipients, binders, disintegrators, lubricants, isotonic agents, stabilizers, and preservatives.

Moreover, the pharmaceutical composition of the present invention can also be in other solid preparations, for example, tablets, pills, granules, powders, oral tablets (troches, sublingual tablets, buccal tablets, adhesive tablets, and gum forms), and capsules.

For creams, for example, the bacterium of the present invention can be used in a cream form by mixing it with an oily phase component containing a solid oil component composed of white petroleum jelly and high-grade alcohols and a liquid oil component composed of squalane, a water phase component, and a surfactant. The oily phase component of the cream can also include other solid and liquid oils in addition to the aforementioned white petroleum jelly, high-grade alcohols, and squalane.

For gels, for example, the bacterium of the present invention can be dissolved in a solvent such as ethanol or polyhydric alcohols with a solubilizing agent and mixed with a gel phase that has been thickened by adding a thickening agent to water to be used as a gel.

For patches, for example, the bacterium of the present invention can be combined with a (solid) carrier or matrix such as a bandage, BAND-AID, or tape to be used as a patch.

For spray preparations (including inhalants), for example, the bacterium of the present invention can be delivered in the form of an aerosol spray from a pressurized container or dispenser containing an appropriate propellant, such as carbon dioxide gas or a nebulizer, to be used as a spray preparation.

There are no limitations on the administration method of the pharmaceutical composition of the present invention, and it may be orally administered or parenterally administered. For oral administration, in addition to oral intake by drinking or eating the pharmaceutical composition of the present invention, a form of applying the pharmaceutical composition inside the oral cavity can be mentioned. For parenteral administration, intravenous injection, transdermal administration, intradermal administration, subcutaneous administration, nasal administration (inhalation from the nose), intraocular administration, intramuscular administration, and intratumoral administration can be mentioned. Among these, oral administration, intravenous injection, transdermal administration, nasal administration, and intratumoral administration are preferable, with intravenous injection, transdermal administration, and intratumoral administration being more preferable.

Furthermore, there are no particular limitations on the dosage and dosage interval, and an appropriate dosage and dosage interval may be selected according to the intended use and patient.

As for the administration target of the pharmaceutical composition of the present invention, mammals can be mentioned, with humans being preferable. The pharmaceutical composition of the present invention can be applied for the treatment and/or prevention of various types of diseases or conditions depending on the type of heterologous protein produced and secreted. Examples of diseases or conditions include central nervous system disorders (such as Alzheimer's disease, Parkinson's disease, and ischemic neuronal damage), inflammatory diseases (such as allergic diseases, asthma, rheumatism, and osteoarthritis), cardiovascular diseases (such as heart failure, cardiac hypertrophy, angina pectoris, and arteriosclerosis), cancer (such as non-small cell lung cancer, ovarian cancer, prostate cancer, stomach cancer, bladder cancer, breast cancer, cervical cancer, colon cancer, and rectal cancer), diabetes, immune system disorders (such as autoimmune diseases, atopic dermatitis, allergic diseases, immunodeficiency, asthma, rheumatoid arthritis, psoriasis, arteriosclerosis, diabetes, and Alzheimer's disease), hepatic injuries and gallbladder diseases (such as liver cirrhosis, hepatitis, liver failure, cholestasis, and gallstones), digestive system disorders (such as ulcers, enteritis, indigestion, irritable bowel syndrome, ulcerative colitis, diarrhea, and ileus), burns, fractures, alopecia, acne, acne-like rash, stomatitis, etc. Among these, central nervous system disorders, inflammatory diseases, cancer, immune system disorders, burns, alopecia, acne, acne-like rash, and stomatitis are preferable, with cancer, immune system disorders, burns, alopecia, acne, acne-like rash, and stomatitis being particularly preferable.

Furthermore, the pharmaceutical composition of the present invention can be suitably used to treat or prevent diseases or conditions selected from cancer, lower limb ischemic diseases, rashes or intraoral mucositis accompanying cancer treatment, ichthyosis, cavities, irritable bowel syndrome, obesity, injuries, and surgical scars. Particularly preferably, the pharmaceutical composition of the present invention can be suitably used to treat or prevent lower limb ischemic diseases.

The fourth embodiment of the present invention is the use of the aforementioned bacterium in the production of medicines. As for medicines and uses thereof, what is described in the section on the pharmaceutical composition above can be mentioned.

### Examples

Hereinafter, the present invention will be specifically described with reference to Examples. However, the present invention is not limited to the Examples described later.
<1> Secretion expression of human-derived basic fibroblast growth factor (bFGF or FGF2) using the signal peptide (TorAss) of trimethylamine N-oxide reductase derived from Escherichia coli by Lactococcus lactis subsp. cremoris MG1363 strain (LMBP 3019) heterologously expressing the Tat system secretion apparatus (CgTatABC) derived from Corynebacterium glutamicum 2256 (ATCC 13869)

### <1-1> Construction of a plasmid vector (pNZ9148) carrying a constant expression promoter PpepN

The promoter region and SD sequence of the pepN gene derived from Lactococcus lactis subsp. cremoris MG1363 strain (SEQ ID NO: 1; hereinafter referred to as PpepN; I. J. van Alen-Boerrigter et. al., Appl. Environ. Microbiol., 57(9), 2555-2561 (1991)) were acquired by artificial gene synthesis (Genscript). This sequence was amplified by PCR using synthetic DNAs of SEQ ID NOs: 2 and 3 as primers. The resulting PCR product and the pNZ8148 vector (manufactured by GoldBio) sequence digested by BglII and PaeI (manufactured by Thermo Fisher Scientific) were linked with each other using In-Fusion Snap Assembly Master Mix (manufactured by Clontech). The resulting DNA was used to transform Escherichia coli MC1061F⁻ strain competent cells (manufactured by Lucigen), smeared on LB agar media containing 10 µg/mL chloramphenicol (tryptone 10 g/L, yeast extract 5 g/L, sodium chloride 5 g/L, and agar 15 g/L), and cultured overnight at 37°C. Plasmids were extracted from the resulting transformants, and those with the target PCR product inserted were named pNZ9148.

### <1-2> Construction of TatABC expression vector derived from Corynebacterium glutamicum (pNZ-PpepN-CgtatAB)

C. glutamicum 2256 strain-derived tatA, tatB, and tatC genes (SEQ ID NOs: 4, 5, and 6; Y. kikuchi et. al., Appl. Environ. Microbial., 72(11), 7138-7192 (2006)) were each optimized for the codon of L. lactis MG1363 strain, and the sequence obtained by operonization of those three genes (SEQ ID NO: 7; hereinafter referred to as CgtatABC) was acquired by artificial gene synthesis (Twist Bioscience). This sequence was amplified by PCR using synthetic DNAs of SEQ ID NOs: 8 and 9 as primers. The resulting PCR product and the pNZ9148 vector sequence digested by SpeI and HindIII (manufactured by Takara) were linked with each other using In-Fusion Snap Assembly Master Mix (manufactured by Clontech). The resulting DNA was used to transform Escherichia coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ9148-CgtatABC.

### <1-3> Construction of TorAss-FGF2 fusion protein expression vector (pNZ9148-torAss-fgf2)

To express Escherichia coli-derived TorA signal peptide and human-derived FGF2 as a fusion protein, the base sequences encoding each of their amino acid sequences (SEQ ID NOs: 27 and 11) were connected, and the ORF sequence optimized for the codons of Lactococcus lactis subsp. cremoris MG1363 strain (SEQ ID NO: 12; hereafter referred to as torAss-fgf2) was acquired by artificial gene synthesis (Twist Bioscience). This sequence was amplified by PCR using synthetic DNAs of SEQ ID NOs: 13 and 14 as primers. The resulting PCR product and the pNZ9148 vector sequence digested by SpeI and HindIII (manufactured by Takara) were linked with each other using In-Fusion Snap Assembly Master Mix (manufactured by Clontech). The resulting DNA was used to transform Escherichia coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ9148-torAss-fgf2.

### <1-4> Construction of CgTatABC and TorAss-FGF2 fusion protein expression vector (pNZ9148-CgtatAB-torAss-fgf2)

A sequence containing the CgtatABC sequence, the pepN promoter sequence, and downstream torA-fgf sequence (CgtatABC-PpepN-torA-fgf2) was acquired by artificial gene synthesis (Twist Bioscience). This sequence was amplified by PCR using synthetic DNAs of SEQ ID NOs: 8 and 14 as primers. The resulting PCR product and the pNZ9148 vector sequence digested by SpeI and HindIII (manufactured by Takara) were linked with each other using In-Fusion Snap Assembly Master Mix (manufactured by Clontech). The resulting DNA was used to transform E. coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ9148-CgtatABC-torAss-fgf2.

### <1-5> Construction of Lactococcus lactis expressing CgTatABC or TorAss-FGF2

Each of pNZ9148, pNZ9148-CgtatABC, pNZ9148-torAss-fgf2, and pNZ9148-CgtatABC-torAss-fgf2 was introduced into competent cells of Lactococcus lactis subsp. cremoris MG1363 strain (manufactured by GoldBio) by electroporation, and smeared on M17 agar medium containing 10 µg/mL chloramphenicol and 5 g/L glucose (manufactured by BD Difco), and was cultured overnight at 30°C. The resulting transformants were respectively named Lactococcus lactis MG1363/pNZ9148 strain, Lactococcus lactis MG1363/pNZ9148-CgtatABC strain, Lactococcus lactis MG1363/pNZ9148-torAss-fgf2 strain, and Lactococcus lactis MG1363/pNZ9148-CgtatABC-torAss-fgf2 strain. These strains were each inoculated into 5 mL of M17 medium containing 10 µg/mL chloramphenicol and 5 g/L glucose, and were stationary cultured overnight at 30°C. The resulting culture solutions were mixed with an equal volume of 30% (v/v) glycerol solution and stored at -80°C.

### <1-6> Production and secretion of FGF2 by Lactococcus lactis MG1363/pNZ9148-CgtatABC-torAss-fgf2 strain

Lactococcus lactis MG1363/pNZ9148 strain, Lactococcus lactis MG1363/pNZ9148-CgtatABC strain, Lactococcus lactis MG 13 6 3 /pNZ 914 8 -toras s -fgf2 strain, and Lactococcus lactis MG1363/pNZ9148-CgtatABC-torAss-fgf2 strain preservation liquids were scraped with a 10-µL-volume loop, each inoculated into 5 mL of M17 (G5M17) medium containing 10 µg/mL chloramphenicol and 5 g/L glucose, and shake-cultured for 20 hours at 30°C under anaerobic conditions using AnaeroPack (registered trademark) (manufactured by Mitsubishi Gas Chemical Company, Inc.). Subsequently, 250 µL of the resulting culture solution was inoculated into 5 mL of M17 (G10M17) medium containing 10 µg/mL chloramphenicol, 10 mg/L catalase (derived from bovine liver), 40 g/L calcium carbonate, and 10 g/L glucose, and shake-cultured for 48 hours at 30°C under anaerobic conditions using AnaeroPack (registered trademark) (manufactured by Mitsubishi Gas Chemical Company, Inc.). Afterward, the resulting culture solution was centrifuged at 7500×g at 4°C for 10 minutes. The supernatant was filtered, and the filtrate was concentrated using an ultrafiltration filter (Amicon (registered trademark) Ultra 3K, manufactured by Merck), obtaining a culture supernatant concentrate. The resulting culture supernatant concentrate was subjected to reducing SDS-PAGE using NuPAGE 10% Bit-Tris Protein Gels (manufactured by Thermo Fisher Scientific) and NuPAGE MOPS-SDS Runnning buffer (manufactured by Thermo Fisher Scientific). Subsequently, it was transferred to a PVDF membrane using iBlot Gel transfer Stacks PVDF, Mini (manufactured by Thermo Fisher Scientific) and iBlot 2 Dry Blotting System (manufactured by Thermo Fisher Scientific). The transferred PVDF membrane was blotted with anti-FGF2 monoclonal antibody (manufactured by Thermo Fisher Scientific) and Goat anti-mouse IgG (H+L) - HRP conjugate (manufactured by Thermo Fisher Scientific) using iBind Western Systems (manufactured by Thermo Fisher Scientific) and I Bind Flex Solution Kit (manufactured by Thermo Fisher Scientific). After blotting, the PVDF membrane was washed with ultrapure water, immersed in ECL Prime Western Blotting Detection Reagent (manufactured by Cytiva) at room temperature for 5 minutes, and detected with ChemiDoc (manufactured by Bio-Rad).

The results are shown in Fig. 1. Surprisingly, only in the Lactococcus lactis MG1363/pNZ9148-CgtatABC-torAss-fgf2 strain was the FGF2 band detected, revealing that despite Lactococcus lactis MG1363 not possessing an endogenous Tat system secretion apparatus, by heterologously expressing the Tat system secretion apparatus derived from Corynebacterium glutamicum, it is possible to secrete and express the desired protein via the Tat system.
<2> Secretion expression of FGF2 using the TorA signal peptide derived from E. coli by L. lactis MG1363 strain (LMBP 3019) heterologously expressing the Tat system secretion apparatus (CgTatABC) derived from C. glutamicum 2256 strain and either the Type I signal peptidase (CgLepB; WO 2020/085511) derived from C. glutamicum 2256 strain or the Type I signal peptidase homologue (LllepB) derived from L. lactis MG1363 strain

### <2-1> Construction of a vector expressing CgTatABC, TorAss-FGF2, and CgLepB (pNZ9148-CgtatABC-torAss-fgf2-CglepB)

The gene CglepB (SEQ ID NO: 64) of Type I signal peptidase derived from C. glutamicum 2256 strain, optimized for codons in L. lactis MG1363 strain, was acquired by artificial gene synthesis (Twist Bioscience). This sequence was used as a template, and the sequence including CglepB was amplified by PCR with synthetic DNA of SEQ ID NOs: 65 and 66 as primers. The resulting PCR product and the linearized pNZ9148-CgtatABC-torAss-fgf2 vector by inverse with synthetic DNA of SEQ ID NOs: 67 and 68 as primers were connected by In-Fusion Snap Assembly Master Mix (manufactured by Clontech). The resulting DNA was used to transform E. coli MC1061F⁻ strain competent cells (manufactured by Lucigen), smeared on LB agar medium containing 10 µg/mL chloramphenicol, and cultured overnight at 37°C. Plasmids were extracted from the resulting transformants, and those with the target PCR product inserted were named pNZ9148-CgtatABC-torAss-fgf2-CglepB.

### <2-2> Construction of a vector expressing TorAss-FGF2 and CgLepB (pNZ9148-torAss-fgf2-CglepB)

As with <2-1>, the CglepB sequence acquired by artificial gene synthesis was used as a template, and the sequence including CglepB was amplified by PCR with synthetic DNA of SEQ ID NOs: 65 and 66 as primers. The resulting PCR product and the linearized pNZ9148-torAss-fgf2 vector by inverse PCR with synthetic DNA of SEQ ID NOs: 67 and 68 as primers were connected by In-Fusion Snap Assembly Master Mix (manufactured by Clontech). The resulting DNA was used to transform E. coli MC1061F⁻ strain competent cells (manufactured by Lucigen), smeared on LB agar medium containing 10 µg/mL chloramphenicol, and cultured overnight at 37°C. Plasmids were extracted from the resulting transformants, and those with the target PCR product inserted were named pNZ9148-torAss-fgf2-CglepB.

### <2-3> Construction of a vector expressing CgTatABC, TorAss-FGF2, and LlLepB (pNZ9148-CgtatABC-torAss-fgf2-LllepB)

The genome of the L. lactis MG1363 strain was used as a template, and the sequence including LllepB was amplified by PCR with synthetic DNA of SEQ ID NOs: 69 and 70 as primers. The resulting PCR product and the linearized pNZ9148-CgtatABC-torAss-fgf2 vector by inverse PCR with synthetic DNA of SEQ ID NOs: 67 and 68 as primers were connected by In-Fusion Snap Assembly Master Mix (manufactured by Clontech). The resulting DNA was used to transform E. coli MC1061F⁻ strain competent cells (manufactured by Lucigen), smeared on LB agar medium containing 10 µg/mL chloramphenicol, and cultured overnight at 37°C. Plasmids were extracted from the resulting transformants, and those with the target PCR product inserted were named pNZ9148-CgtatABC-torAss-fgf2-LllepB.

### <2-4> Construction of a vector expressing TorAss-FGF2 and LlLepB (pNZ9148-torAss-fgf2-LllepB)

As with <2-3>, the genome of the L. lactis MG1363 strain was used as a template, and the sequence including LllepB was amplified by PCR with synthetic DNA of SEQ ID NOs: 69 and 70 as primers. The resulting PCR product and the linearized pNZ9148-torAss-fgf2 by PCR with SEQ ID NOs: 67 and 68 were connected by In-Fusion Snap Assembly Master Mix (manufactured by Clontech). The resulting DNA was used to transform E. coli MC1061F⁻ strain competent cells (manufactured by Lucigen), smeared on LB agar medium containing 10 µg/mL chloramphenicol, and cultured overnight at 37°C. Plasmids were extracted from the resulting transformants, and those with the target PCR product inserted were named pNZ9148-torAss-fgf2-LllepB.

### <2-5> Construction of Lactococcus lactis expressing CgTatABC, TorAss-FGF2, and CglepB or LllepB

Each of pNZ9148-CgtatABC-torAss-fgf2-CglepB, pNZ9148-torAss-fgf2-CglepB, pNZ9148-CgtatABC-torAss-fgf2-LllepB, and pNZ9148-torAss-fgf2-LllepB was introduced into competent cells of Lactococcus lactis subsp. cremoris MG1363 strain (manufactured by GoldBio) by electroporation, and smeared on M17 agar medium containing 10 µg/mL chloramphenicol and 5 g/L glucose (manufactured by BD Difco), and was cultured overnight at 30°C. The resulting transformants were respectively named Lactococcus lactis MG1363/pNZ9148-CgtatABC-torAss-fgf2-CglelpB strain, Lactococcus lactis MG 13 6 3 /pNZ 914 8 -toras s -fgf2 -Cgl epB strain, Lactococcus lactis MG1363/pNZ9148-CgtatABC-torAss-fgf2-LllepB strain, and Lactococcus lactis MG1363/pNZ9148-torAss-fgf2-LllepB strain. These strains were each inoculated into 5 mL of M17 medium containing 10 µg/mL chloramphenicol and 5 g/L glucose, and were stationary cultured overnight at 30°C. The resulting culture solutions were mixed with an equal volume of 30% (v/v) glycerol solution and stored at -80°C.

### <2-6> Improved production and secretion of mature FGF2 by Lactococcus lactis MG1363/pNZ9148-CgtatABC-torAss-fgf2-CglepB strain and Lactococcus lactis MG1363/pNZ9148-CgtatABC-torAss-fgf2-LllepB strain

Lactococcus lactis MG1363/pNZ9148 strain, Lactococcus lactis MG1363/pNZ9148-CgtatABC strain, Lactococcus lactis MG 13 6 3 /pNZ 914 8 -toras s -fgf2 strain, Lactococcus lactis MG1363/pNZ9148-CgtatABC-torAss-fgf2 strain, Lactococcus lactis MG1363/pNZ9148-CgtatABC-torAss-fgf2-CglelpB strain, Lactococcus lactis MG 13 6 3 /pNZ 914 8 -toras s -fgf2 -Cgl epB strain, Lactococcus lactis MG1363/pNZ9148-CgtatABC-torAss-fgf2-LllepB strain, and Lactococcus lactis MG1363/pNZ9148-torAss-fgf2-LllepB strain were scraped with a 10-µL-volume loop, each inoculated into 5 mL of M17 (G5M17) medium containing 10 µg/mL chloramphenicol and 5 g/L glucose, and shake-cultured for 20 hours at 30°C under anaerobic conditions using AnaeroPack (registered trademark) (manufactured by Mitsubishi Gas Chemical Company, Inc.). Subsequently, 250 µL of the resulting culture solution was inoculated into 5 mL of M17 (G10M17) medium containing 10 µg/mL chloramphenicol, 10 mg/L catalase (derived from bovine liver), 40 g/L calcium carbonate, and 10 g/L glucose, and shake-cultured for 48 hours at 30°C under anaerobic conditions using AnaeroPack (registered trademark) (manufactured by Mitsubishi Gas Chemical Company, Inc.). Afterward, the resulting culture solution was centrifuged at 7500×g at 4°C for 10 minutes. The supernatant was filtered, and the filtrate was concentrated using an ultrafiltration filter (Amicon (registered trademark) Ultra 3K, manufactured by Merck), obtaining a culture supernatant concentrate. The protein concentration in the resulting culture supernatant concentrate was quantified using the Quick Start Bradford protein assay kit (manufactured by Bio-Rad). The culture supernatant was diluted so that the total protein amount was 12 µg, and it was subjected to reducing SDS-PAGE using NuPAGE 10% Bit-Tris Protein Gels (manufactured by Thermo Fisher Scientific) and NuPAGE MOPS-SDS Runnning buffer (manufactured by Thermo Fisher Scientific). Subsequently, it was transferred to a PVDF membrane using iBlot Gel transfer Stacks PVDF, Mini (manufactured by Thermo Fisher Scientific) and iBlot 2 Dry Blotting System (manufactured by Thermo Fisher Scientific). The transferred PVDF membrane was blotted with anti-FGF2 monoclonal antibody (manufactured by Thermo Fisher Scientific) and Goat anti-mouse IgG (H+L) - HRP conjugate (manufactured by Thermo Fisher Scientific) using iBind Western Systems (manufactured by Thermo Fisher Scientific) and I Bind Flex Solution Kit (manufactured by Thermo Fisher Scientific). After blotting, the PVDF membrane was washed with ultrapure water, immersed in ECL Prime Western Blotting Detection Reagent (manufactured by Cytiva) at room temperature for 5 minutes, and detected with ChemiDoc (manufactured by Bio-Rad). The bands were quantified using the software Quantity One (manufactured by Bio-rad).

The results are shown in Fig. 2. In the Lactococcus lactis MG1363/pNZ9148-CgtatABC-torAss-fgf2-LllepB strain and the Lactococcus lactis MG1363/pNZ9148-CgtatABC-torAss-fgf2-CglepB strain, compared to Lactococcus lactis MG1363/pNZ9148-CgtatABC-torAss-fgf2, the amount of mature FGF2 secreted, with the signal sequence removed, increased by an average of 1.9 times and 1.5 times, respectively. Therefore, it has been demonstrated that the heterologous expression of signal peptidase I derived from Corynebacterium glutamicum or the enhancement of signal peptidase I derived from Lactococcus lactis improves the secretion ability of mature protein by Lactococcus lactis MG1363 heterologously expressing the Tat system secretion apparatus derived from Corynebacterium glutamicum.
<3> Secretion expression of Pro-PGHisx6 using the TorA signal peptide derived from E. coli by L. lactis MG1363 strain (LMBP 3019) heterologously expressing the Tat system secretion apparatus (CgTatABC) derived from C. glutamicum 2256 strain and either the Type I signal peptidase homolog (LllepB) derived from L. lactis MG1363 strain

### <3-1> Construction of a vector expressing TorAss-Pro-PGHisx6 and LllepB (pNZ9148-torAss-ppghis-LllepB)

A gene sequence of protein glutaminase derived from Chryseobacterium proteolyticum, including a pro sequence at the N-terminus and with a polyhistidine tag added to the C-terminus (Pro-PGHisx6), was codon-optimized for Lactococcus lactis MG1363 strain (SEQ ID NO: 85) and acquired by artificial gene synthesis (Twist Bioscience). The resulting sequence was amplified by PCR using synthetic DNAs of SEQ ID NOs: 86 and 87 as primers. The resulting sequence and the linearized pNZ9148-torAss-fgf2-LllepB vector, excluding the fgf2 sequence, by PCR with synthetic DNAs of SEQ ID NOs: 88 and 89 as primers, were connected by In-Fusion Snap Assembly Master Mix (manufactured by Clontech). The resulting DNA was used to transform E. coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ 914 8 -tor As s-ppghi s-Lll epB.

### <3-2> Construction of a vector expressing CgTatABC, TorAss-Pro-PGHisx6, and LlLepB (pNZ9148-Pusp45-CgtatABC-torAss-ppghis-LllepB)

As with <3-1>, the gene sequence of Pro-PG amplified by PCR using synthetic DNAs of SEQ ID NOs: 86 and 87 as primers and the linearized pNZ9148-CgtatABC-torAss-fgf2-LllepB vector, excluding the fgf2 sequence, by PCR with synthetic DNAs of SEQ ID NOs: 88 and 89 as primers, were connected by In-Fusion Snap Assembly Master Mix (manufactured by Clontech). The resulting DNA was used to transform E. coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ9148-CgtatABC-torAss-ppghis-LllepB. To modify the CgtatABC expression promoter, the genome of L. lactis MG1363 strain was used as a template, and by PCR using synthetic DNAs of SEQ ID NOs: 90 and 91 as primers, the sequence including the usp45 primer (SEQ ID NO: 58) was amplified. This and the linearized pNZ9148-CgtatABC-torAss-ppghis-LllepB vector, excluding the pepN promoter sequence, by PCR with synthetic DNAs of SEQ ID NOs: 92 and 93 as primers, were connected by In-Fusion Snap Assembly Master Mix (manufactured by Clontech). The resulting DNA was used to transform E. coli MC1061F⁻ strain competent cells (manufactured by Lucigen), smeared on LB agar media containing 10 µg/mL chloramphenicol, and cultured overnight at 37°C. Plasmids were extracted from the resulting transformants, and those with the target PCR product inserted were named pNZ-Pusp45-CgtatABC-torAss-ppghis-LllepB.

### <3-3> Construction of Lactococcus lacits MG1363 strain expressing CgtatABC, TorAss-pro-PG, and LllepB

Each of pNZ9148-torAss-ppg-LllepB and pNZ9148-Pusp45-CgtatABC-torAss-ppghis-LllepB was introduced into competent cells of Lactococcus lactis subsp. cremoris MG1363 strain (manufactured by GoldBio) by electroporation, and smeared on M17 agar medium containing 10 µg/mL chloramphenicol and 5 g/L glucose (manufactured by BD Difco), and was cultured overnight at 30°C. The resulting transformants were respectively named Lactococcus lactis MG1363/pNZ9148-torAss-ppghis-LllepB strain and Lactococcus lactis MG1363/pNZ9148-Pusp45-CgtatABC-torAss-ppghis-LllepB strain. These strains were each inoculated into 5 mL of M17 medium containing 10 µg/mL chloramphenicol and 5 g/L glucose, and were stationary cultured overnight at 30°C. The resulting culture solutions were mixed with an equal volume of 30% (v/v) glycerol solution and stored at -80°C.

### <3-4> Improved production and secretion of mature Pro-PGHisx6 by Lactococcus lactis MG1363/pNZ9148-Pusp45-CgtatABC-torAss-ppghis-LllepB strain

Lactococcus lactis MG1363/pNZ9148-torAss-ppghis-LllepB strain and Lactococcus lactis MG1363/pNZ9148-Pusp45-CgtatABC-torAss-ppghis-LllepB strain were scraped with a 1-µL-volume loop, each inoculated into 5 mL of M17 (G5M17) medium containing 10 µg/mL chloramphenicol and 5 g/L glucose, and shake-cultured for 20 hours at 30°C under anaerobic conditions using AnaeroPack (registered trademark) (manufactured by Mitsubishi Gas Chemical Company, Inc.). Subsequently, 100 µL of the resulting culture solution was inoculated into 5 mL of M17 (G10M17) medium containing 10 µg/mL chloramphenicol, 10 mg/L catalase (derived from bovine liver), 40 g/L calcium carbonate, and 10 g/L glucose, and shake-cultured for 48 hours at 30°C under anaerobic conditions using AnaeroPack (registered trademark) (manufactured by Mitsubishi Gas Chemical Company, Inc.). Afterward, the resulting culture solution was centrifuged at 7500×g at 4°C for 10 minutes. The supernatant was filtered, and the filtrate was concentrated using an ultrafiltration filter (Amicon (registered trademark) Ultra 3K, manufactured by Merck), obtaining a culture supernatant concentrate. The protein concentration in the resulting culture supernatant concentrate was quantified using the Quick Start Bradford protein assay kit (manufactured by Bio-Rad). The culture supernatant was diluted so that the total protein amount was 18 µg, and it was subjected to reducing SDS-PAGE in the same method as in <2-6>. Subsequently, it was transferred to a PVDF membrane in the same method as in <2-6>, and was blotted with a 6x-His tag monoclonal antibody (His. H8) and Goat anti-mouse IgG (H+L) - HRP conjugate (manufactured by Thermo Fisher Scientific). After blotting, the PVDF membrane was washed with ultrapure water, immersed in ECL Prime Western Blotting Detection Reagent (manufactured by Cytiva) at room temperature for 5 minutes, and detected with ChemiDoc (manufactured by Bio-Rad). The molecular weights were estimated using the software Quantity One (manufactured by Bio-rad).

The results are shown in Fig. 3. A band was detected only in Lactococcus lactis MG1363/pNZ9148-Pusp45-CgtatABC-torAss-ppghis-LllepB strain, around 33.5 kDa, which is the estimated molecular weight of Pro-PGHisx6. Additionally, no band was detected around 43.3 kDa, which is the molecular weight of the induction protein TorAss-Pro-PGHisx6 with the signal sequence uncut. Therefore, it has been demonstrated that the enhancement of signal peptidase I derived from Lactococcus lactis and the heterologous expression of the Tat system secretion apparatus derived from Corynebacterium glutamicum leads to the secretion of Pro-PGHisx6 with the signal sequence removed.
<4> Secretion expression of FGF2 using the TorA signal peptide derived from E. coli or the FAD-dependent oxidoreductase signal peptide derived from Bifidobacterium longum BIOML-A18 strain by Bifidobacterium longum 105-A strain (JCM 31944) heterologously expressing the Tat system secretion apparatus (CgTatABC) derived from C. glutamicum 2256 strain or the Tat system secretion apparatus (BlTatABC) derived from Bifidobacterium longum E-18 strain
<5> Secretion expression of FGF2 using the TorA signal peptide derived from E. coli by Staphylococcus epidermidis ATCC 12228 strain heterologously expressing the Tat system secretion apparatus (CgTatABC) derived from C. glutamicum 2256 strain or the Tat system secretion apparatus (BsTatAdCd) derived from Bacillus subtilis 168 strain
<6> Secretion expression of FGF2 using the TorA signal peptide derived from E. coli by Lactobacillus reuteri DSM20016 strain heterologously expressing the Tat system secretion apparatus (CgTatABC) derived from C. glutamicum 2256 strain
<6-1> Construction of Lactobacillus reuteri DSM20016 strain expressing CgTatABC or TorAss-FGF2

Competent cells of Lactobacillus reuteri DSM20016 were prepared using the method described in the existing report (Holo, H. and Nes, I. N., 1995. Mehotds. Mol. Biol. 47: 195-199). A glycerol stock of Lb. reuteri DSM20016 strain was scraped with a 1-µL-volume loop, smeared MRS (MRSCS) agar medium (manufactured by BD Difco) containing 0.22% (w/v) L-cysteine hydrochloride monohydrate and 3.4% (w/v) sodium L-ascorbate, and cultured overnight at 37°C under anaerobic conditions using AnaeroPack (registered trademark) (manufactured by Mitsubishi Gas Chemical Company, Inc.). The resulting bacterial bodies were scraped with approximately half of a 10-µL-volume loop and inoculated into 40 mL of MRSCS medium containing 0.5 M sucrose and 2% (w/v) glycine, and were static cultured until the OD660 of the culture solution reached 0.5 to 0.6. The resulting culture solution was centrifuged at 7500×g at 4°C for 10 minutes using a large centrifuge, and the supernatant was discarded. The resulting bacterial body pellets were washed three times with 20 mL of ice-cold Washing solution (0.5 M sucrose, 10% (v/v) glycerol), then suspended in 1.8 mL of ice-cold 10% (v/v) glycerol, and centrifuged at 16,200×g at 4°C for 10 minutes using a small centrifuge. The supernatant was discarded, and the resulting bacterial body pellets were resuspended in 1.8 mL of 10% (v/v) glycerol, aliquoted into 60 µL portions, and stored at -80°C.

Each of the plasmid vectors pNZ9148, pNZ9149-CgtatABC, pNZ9148-torAss-fgf2, and pNZ9148-CgtatABC-torAss-fgf2, prepared in <1-1>, <1-2>, <1-3>, and <1-4>, respectively, was introduced into competent cells of Lactobacillus reuteri DSM20016 by electroporation, and smeared on MRSCS agar medium containing 10 µg/mL chloramphenicol, and was cultured overnight at 37°C under anaerobic conditions using AnaeroPack (registered trademark) (manufactured by Mitsubishi Gas Chemical Company, Inc.). The resulting transformants were respectively named Lactobacillus reuteri DSM20016/pNZ9148 strain, Lactobacillus reuteri DSM20016/pNZ9148-CgtatABC strain, Lactobacillus reuteri DSM20016/pNZ9148-torAss-fgf2 strain, and Lactobacillus reuteri DSM20016/pNZ9148-CgtatABC-torAss-fgf2 strain. These strains were each inoculated into 5 mL of MRSCS medium containing 10 µg/mL chloramphenicol, and were statically cultured overnight at 37°C under anaerobic conditions using AnaeroPack (registered trademark) (manufactured by Mitsubishi Gas Chemical Company, Inc.). The resulting culture solutions were mixed with an equal volume of 30% (v/v) glycerol solution and stored at -80°C.

### <6-2> FGF2 production and secretion by Lactobacillus reuteri DSM20016/pNZ9148-CgtatABC-torAss-fgf2 strain

Glycerol stocks of Lactobacillus reuteri DSM20016/pNZ9148 strain, Lactobacillus reuteri DSM20016/pNZ9148-CgtatABC strain, Lactobacillus reuteri DSM20016/pNZ9148-torAss-fgf2 strain, and Lactobacillus reuteri DSM20016/pNZ9148-CgtatABC-torAss-fgf2 strain were scraped with a 10-µL-volume loop, each inoculated into 5 mL of MRSCS medium containing 10 µg/mL chloramphenicol, and shake-cultured for 20 hours at 37°C under anaerobic conditions using AnaeroPack (registered trademark) (manufactured by Mitsubishi Gas Chemical Company, Inc.). Subsequently, 100 µL of the resulting culture solution was inoculated into 5 mL of MRSCS medium containing 10 µg/mL chloramphenicol, 40 g/L calcium carbonate, and 10 mg/L catalase, and shake-cultured for 48 hours at 37°C under anaerobic conditions using AnaeroPack (registered trademark) (manufactured by Mitsubishi Gas Chemical Company, Inc.). Afterward, the resulting culture solution was centrifuged at 7500×g at 4°C for 10 minutes. The supernatant was filtered, and the filtrate was concentrated using an ultrafiltration filter (Amicon (registered trademark) Ultra 3K, manufactured by Merck), obtaining a culture supernatant concentrate. The resulting culture supernatant concentrate was subjected to Western blotting in the same manner as <1-6>.

The results are shown in Fig. 4. Only in the Lactobacillus reuteri DSM20016/pNZ9148-CgtatABC-torAss-fgf2 strain was the FGF2 band detected, revealing that despite the Lactobacillus reuteri DSM20016 strain not possessing an endogenous Tat system secretion apparatus, by heterologously expressing the Tat system secretion apparatus derived from Corynebacterium glutamicum, it is possible to secrete and express the desired protein via the Tat system.

**Table 1**

| SEQ ID NO | Sequence |
|---|---|
| 1 | CTCTGTAAAAGCTGTCAGTAGACAGTTTTTTTAATAAGTTAAAGAAAAGAT GTAATTTTTCTTTGTACTCGAAATTTTCTATTCAATTTGATATAATTATATTAAT ACTGAATATTTAGGAGAAG |
| 2 | TTACAGCTCCAGATCTCTCTGTAAAAGCTGTCAGTA |
| 3 | GAACTAGTGGTACCGCATGCCTTCTCCTAAATATTCAGTA |
| 4 | ATGCCCACTCTCGGACCATGGGAAATCGCGATCATTGTCCTGCTGATCATTC TGCTGTTCGGCGCGAAGAAGCTGCCTGATGCAGCTCGTTCCATCGGCCGTT CCATGCGCATCTTCAAGTCTGAAGTCAAAGAAATGAACAAGGACGGCGAT ACCCCAGAACAACAACAGCAGCAGCCTCAGCAGCAGCAGCAGATTGCGC CCAACCAGATCGAGGCTCCTCAGCCAGTTCAGCAGCCAGCGCAACAGTCA AACTTTGAGCAGCACTACCAGGGCCAGCAGGTTCAGCAGCCTCAGAACCC TCAGACCCCTGACTACCGTCAGAACTACGAGGATCCAAACCGCACCTCCT AA |
| 5 | ATGTTTTCTAGCGTGGGTTGGGGAGAGATCTTCCTCTTAGTCGTTGTGGGC CTTGTTGTCATCGGCCCGGAACGGTTGCCTCGTTTGATCCAGGACGCACGC GCTGCGCTGCTCGCTGCACGTACCGCTATCGACAATGCAAAGCAGTCGTTG GACAGTGATTTTGGTTCGGAATTTGATGAAATCCGAAAGCCACTAACCCAG |
| | GTTGCACAGTACAGCCGGATGAGCCCCAAGACGGCCATCACTAAGGCGTT GTTTGATAATGATTCCTCGTTCCTGGATGACTTTGATCCAAAGAAGATCATG GCCGAAGGAACAGAAGGCGAAGCTCAGCGCCACAAGCAGGCAGCTGACA ACAATGCGAATGTGGTGGAACGTCCAGCTGATGGTTCCACCGCACGCCCA ACGCAAAACGATCCAAAAGACGGCCCGAATTACTCAGGCGGCGTCTCTTG GACCGATATTATTTAG |
| 6 | ATGTCCATTGTTGAGCACATCAAAGAGTTTCGACGCCGACTTCTCATCGCT CTGGCGGGCATCCTCGTGGGCACCATTATCGGCTTTATTTGGTACGATTTCT CATTTTGGCAGATCCCCACTTTGGGCGAGCTGCTGAGGGATCCGTACTGTT CTTTGCCTGCTGAATCCCGCTGGGCCATGAGCGACTCAGAGGAATGTCGA CTGCTCGCAACCGGCCCGTTTGATCCATTCATGCTTCGCCTTAAAGTAGCG GCGTTGGTGGGTATGGTTCTTGGCTCACCCGTGTGGCTGAGCCAGCTGTGG GGCTTTATCACCCCAGGTTTGATGAAGAATGAGCGCCGTTACACCGCAATC TTCGTCACGATTGCTGTTGTGCTGTTTGTCGGCGGTGCTGTTCTTGCGTACT TCGTCGTTGCATATGGTTTGGAGTTCCTCCTTACCATTGGTGGAGACACCC AGGCAGCGGCCCTGACTGGTGATAAGTACTTCGGATTCTTGCTCGCGTTGT TGGCGATTTTCGGCGTGAGCTTCGAAGTTCCACTGGTGATCGGCATGCTCA ACATTGTGGGTATCTTGCCCTACGATGCCATTAAAGATAAGCGACGCATGAT CATCATGATTTTGTTCGTGTTCGCTGCTTTCATGACACCCGGCCAGGATCCT TTCACCATGTTGGTGTTGGCGCTTTCACTCACCGTTCTGGTGGAGCTTGCC CTGCAGTTCTGTCGCTTCAACGACAAACGCCGGGACAAGAAGCGCCCAG AATGGCTTGATGGCGATGACCTCTCTGCATCACCACTGGATACTTCTGCTG GTGGAGAAGATGCTCCAAGCCCAGTCGAAACCCCAGAGGCGGTGGAGCC TTCGCGGATGCTGAACCCAAGTGGGGAGGCGTCGATAAGCTATAAACCCG GGCGCGCCGACTTCGGTGACGTGCTCTAG |
| 7 | ATGCCAACACTTGGTCCATGGGAAATTGCTATTATTGTATTATTGATTATTTT ACTTTTTGGCGCAAAAAAATTGCCAGATGCTGCGCGTTCAATTGGACGAA GTATGCGTATCTTTAAAAGTGAGGTAAAGGAAATGAATAAAGATGGTGACA CACCTGAACAACAACAACAACAACCACAACAACAACAACAAATTGCACC |
| | AAATCAAATTGAGGCTCCTCAACCAGTACAACAGCCTGCTCAACAAAGTA ATTTTGAACAACATTATCAAGGCCAACAAGTCCAACAACCACAAAACCCA CAAACGCCAGATTATCGTCAAAATTATGAAGACCCAAATCGAACGTCATAA AGGAGAAGGCATGAGTATTGTGGAACATATTAAAGAGTTTCGACGCCGTTT ACTTATTGCACTTGCGGGGATTCTTGTTGGAACGATTATCGGATTCATCTGG TATGATTTTAGTTTCTGGCAAATTCCTACATTGGGAGAGTTACTTCGTGACC CATATTGTAGTCTTCCTGCCGAGTCACGCTGGGCTATGTCTGATTCAGAAG AATGCCGTTTATTAGCAACCGGGCCATTTGATCCTTTCATGTTACGTTTGAA AGTGGCTGCACTTGTAGGCATGGTTTTGGGCTCTCCAGTCTGGTTATCTCA ATTATGGGGTTTTATCACTCCAGGTTTGATGAAAAATGAACGTCGTTACACT GCAATATTTGTTACAATTGCAGTTGTCTTATTTGTGGGAGGAGCGGTATTAG CATATTTTGTCGTAGCATATGGTTTAGAATTTCTTCTTACTATCGGTGGAGAT ACACAAGCGGCGGCATTAACGGGAGATAAATATTTTGGTTTCTTGCTTGCT TTGCTTGCTATTTTTGGCGTGAGTTTTGAAGTACCTCTTGTTATTGGGATGT TGAATATTGTCGGAATCTTACCATATGACGCAATTAAAGATAAACGTCGAAT GATTATCATGATATTGTTCGTATTTGCCGCATTTATGACTCCAGGACAGGATC CTTTTACTATGTTGGTTTTAGCTTTATCATTAACGGTTCTTGTTGAATTGGCA TTACAATTCTGTCGATTTAATGATAAACGTCGTGATAAGAAAAGACCTGAG TGGCTTGATGGTGATGATCTTAGTGCATCACCACTTGACACCTCAGCTGGA GGAGAGGATGCACCATCTCCTGTTGAAACGCCAGAAGCAGTAGAACCTTC ACGAATGTTAAATCCATCTGGAGAAGCATCTATATCATACAAACCTGGTCGT GCTGATTTCGGTGATGTTCTTTGAAGGAGAAGGCATGTTTTCATCTGTGGG ATGGGGTGAAATTTTCCTTCTTGTTGTAGTAGGTCTTGTAGTAATTGGACCA GAACGCCTTCCACGCCTTATCCAGGATGCGCGTGCAGCTTTGTTAGCCGCA CGTACAGCTATTGACAATGCCAAACAATCATTAGATTCTGATTTCGGATCAG AGTTCGATGAAATTAGAAAACCACTTACACAAGTTGCTCAATATTCTAGAA TGTCTCCAAAAACAGCAATAACTAAAGCCTTATTTGACAATGATTCTTCATT TTTAGACGATTTTGACCCAAAGAAAATTATGGCAGAAGGCACCGAAGGAG AAGCACAACGTCATAAACAAGCAGCAGACAATAATGCAAATGTCGTAGAA |
| | CGCCCTGCAGACGGTAGTACAGCTCGTCCTACACAAAACGATCCAAAAGA TGGCCCAAATTATAGTGGGGGTGTTTCATGGACTGATATCATTTAA |
| 8 | ACTAGTATGCCAACACTTGGTCCATGGGAAA |
| 9 | AAGCTTTTAAATGATATCAGTCCATGAAACA |
| 10 | ATGAACAATAACGATCTCTTTCAGGCATCACGTCGGCGTTTTCTGGCACAA CTCGGCGGCTTAACCGTCGCCGGGATGCTGGGGCCGTCATTGTTAACGCCG CGACGTGCGACTGCG |
| 11 | ATGGCTGCCGGATCTATTACCACGCTCCCGGCGCTCCCGGAAGACGGTGGA AGCGGCGCCTTCCCACCTGGCCACTTCAAAGACCCGAAACGTCTGTATTGT AAAAATGGCGGTTTTTTCCTCCGCATCCACCCAGATGGTCGCGTCGATGGT GTCCGCGAAAAGTCCGACCCACACATCAAGCTGCAACTGCAAGCAGAGG AACGTGGCGTCGTGAGCATCAAGGGCGTGTGCGCAAACCGCTATCTGGCC ATGAAGGAGGATGGTCGCCTTCTTGCATCCAAATGCGTGACCGATGAATGC TTCTTCTTTGAACGGCTGGAGTCCAACAATTACAACACCTACCGCTCCCGC AAGTATACCTCCTGGTATGTCGCCCTGAAACGCACGGGACAATATAAACTG GGTTCCAAGACCGGTCCTGGCCAAAAGGCGATTCTTTTCCTCCCAATGTCC GCGAAGTCTTGA |
| 12 | ATGAATAATAATGATTTATTTCAAGCATCTAGACGACGATTTTTGGCTCAATT AGGCGGTTTGACAGTTGCTGGGATGTTGGGTCCTTCATTATTGACTCCTCG TCGTGCCACCGCAGCAGCAGGAAGTATCACCACTCTTCCTGCATTACCTGA GGATGGTGGATCTGGAGCTTTTCCACCAGGACATTTTAAAGATCCAAAACG TTTGTATTGTAAAAACGGAGGATTTTTTTTACGTATCCATCCAGACGGACGT GTCGATGGAGTTCGAGAGAAGTCAGATCCACATATTAAATTACAATTACAG GCAGAGGAAAGAGGAGTTGTTTCTATTAAAGGAGTTTGTGCAAATCGTTAT TTAGCTATGAAAGAAGATGGTCGTTTACTTGCTTCTAAATGTGTCACTGAC GAATGTTTCTTCTTTGAAAGATTGGAATCTAATAATTACAATACCTATCGTTC ACGTAAATATACTTCATGGTATGTTGCTTTAAAACGCACTGGTCAATATAAA CTTGGGTCTAAGACAGGTCCTGGGCAAAAAGCTATTTTATTTCTTCCAATG TCAGCGAAAAGTTAA |
| 13 | ACTAGTATGAATAATAATGATTTATTTCAAG |
| 14 | AAGCTTTTAACTTTTCGCTGACATTGGAAGA |
| 15 | ATGCCAACACTTGGTCCATGGGAAATTGCTATTATTGTATTATTGATTATTTT ACTTTTTGGCGCAAAAAAATTGCCAGATGCTGCGCGTTCAATTGGACGAA GTATGCGTATCTTTAAAAGTGAGGTAAAGGAAATGAATAAAGATGGTGACA CACCTGAACAACAACAACAACAACCACAACAACAACAACAAATTGCACC AAATCAAATTGAGGCTCCTCAACCAGTACAACAGCCTGCTCAACAAAGTA ATTTTGAACAACATTATCAAGGCCAACAAGTCCAACAACCACAAAACCCA CAAACGCCAGATTATCGTCAAAATTATGAAGACCCAAATCGAACGTCATAA |
| 16 | ATGTTTTCATCTGTGGGATGGGGTGAAATTTTCCTTCTTGTTGTAGTAGGTC TTGTAGTAATTGGACCAGAACGCCTTCCACGCCTTATCCAGGATGCGCGTG CAGCTTTGTTAGCCGCACGTACAGCTATTGACAATGCCAAACAATCATTAG ATTCTGATTTCGGATCAGAGTTCGATGAAATTAGAAAACCACTTACACAAG TTGCTCAATATTCTAGAATGTCTCCAAAAACAGCAATAACTAAAGCCTTATT TGACAATGATTCTTCATTTTTAGACGATTTTGACCCAAAGAAAATTATGGCA GAAGGCACCGAAGGAGAAGCACAACGTCATAAACAAGCAGCAGACAATA ATGCAAATGTCGTAGAACGCCCTGCAGACGGTAGTACAGCTCGTCCTACAC AAAACGATCCAAAAGATGGCCCAAATTATAGTGGGGGTGTTTCATGGACTG ATATCATTTAA |
| 17 | ATGAGTATTGTGGAACATATTAAAGAGTTTCGACGCCGTTTACTTATTGCAC TTGCGGGGATTCTTGTTGGAACGATTATCGGATTCATCTGGTATGATTTTAG TTTCTGGCAAATTCCTACATTGGGAGAGTTACTTCGTGACCCATATTGTAGT CTTCCTGCCGAGTCACGCTGGGCTATGTCTGATTCAGAAGAATGCCGTTTA TTAGCAACCGGGCCATTTGATCCTTTCATGTTACGTTTGAAAGTGGCTGCA CTTGTAGGCATGGTTTTGGGCTCTCCAGTCTGGTTATCTCAATTATGGGGTT TTATCACTCCAGGTTTGATGAAAAATGAACGTCGTTACACTGCAATATTTGT TACAATTGCAGTTGTCTTATTTGTGGGAGGAGCGGTATTAGCATATTTTGTC GTAGCATATGGTTTAGAATTTCTTCTTACTATCGGTGGAGATACACAAGCGG CGGCATTAACGGGAGATAAATATTTTGGTTTCTTGCTTGCTTTGCTTGCTATT |
| | TTTGGCGTGAGTTTTGAAGTACCTCTTGTTATTGGGATGTTGAATATTGTCG GAATCTTACCATATGACGCAATTAAAGATAAACGTCGAATGATTATCATGAT ATTGTTCGTATTTGCCGCATTTATGACTCCAGGACAGGATCCTTTTACTATGT TGGTTTTAGCTTTATCATTAACGGTTCTTGTTGAATTGGCATTACAATTCTGT CGATTTAATGATAAACGTCGTGATAAGAAAAGACCTGAGTGGCTTGATGGT GATGATCTTAGTGCATCACCACTTGACACCTCAGCTGGAGGAGAGGATGCA CCATCTCCTGTTGAAACGCCAGAAGCAGTAGAACCTTCACGAATGTTAAAT CCATCTGGAGAAGCATCTATATCATACAAACCTGGTCGTGCTGATTTCGGTG ATGTTCTTTGA |
| 18 | ATGCCGACCCTGGGCCCGTGGGAAATCGCCATCATCGTGCTGCTGATCATC CTGCTGTTCGGCGCCAAGAAGCTGCCGGACGCCGCCCGCTCCATCGGCCG CTCCATGCGCATCTTCAAGTCCGAAGTGAAGGAGATGAACAAGGACGGCG ACACCCCGGAACAGCAGCAGCAGCAGCCGCAGCAGCAGCAGCAGATCGC CCCGAACCAGATCGAAGCCCCGCAGCCGGTGCAGCAGCCGGCCCAGCAG TCCAACTTCGAGCAGCACTACCAGGGCCAGCAGGTGCAGCAGCCGCAGA ACCCGCAGACCCCGGACTACCGCCAGAACTACGAGGACCCGAACCGCAC CTCCTGA |
| 19 | ATGTTCTCCTCCGTGGGCTGGGGCGAAATCTTCCTGCTAGTGGTGGTGGGC CTGGTGGTGATCGGCCCGGAACGCCTGCCGCGCCTGATCCAGGACGCCCG CGCCGCCCTGCTGGCCGCCCGCACCGCCATCGACAACGCCAAGCAGTCCC TGGACTCCGACTTCGGCTCCGAGTTCGACGAAATCCGCAAGCCGCTGACC CAGGTGGCCCAGTACTCCCGCATGTCCCCGAAGACCGCCATCACCAAGGC CCTGTTCGACAACGACTCCTCCTTCCTGGACGACTTCGACCCGAAGAAGA TCATGGCCGAAGGCACCGAGGGCGAAGCCCAGCGCCACAAGCAGGCCGC CGACAACAACGCCAACGTGGTGGAACGCCCGGCCGACGGCTCCACCGCC CGCCCGACCCAGAACGACCCGAAGGACGGCCCGAACTACTCCGGCGGCG TGTCCTGGACCGACATCATCTGA |
| 20 | ATGTCCATCGTGGAACACATCAAGGAGTTCCGCCGCCGCCTGCTGATCGCC CTGGCCGGCATCCTGGTGGGCACCATCATCGGCTTCATCTGGTACGACTTC |
| | TCCTTCTGGCAGATCCCGACCCTGGGCGAACTGCTGCGCGACCCGTACTG CTCCCTGCCGGCCGAATCCCGCTGGGCCATGTCCGACTCCGAGGAATGCC GCCTGCTGGCCACCGGCCCGTTCGACCCGTTCATGCTGCGCCTGAAGGTG GCCGCCCTGGTGGGCATGGTGCTGGGCTCCCCGGTGTGGCTGTCCCAGCT GTGGGGCTTCATCACCCCGGGCCTGATGAAGAACGAACGCCGCTACACCG CCATCTTCGTGACCATCGCCGTGGTGCTGTTCGTGGGCGGCGCCGTGCTGG CCTACTTCGTGGTGGCCTACGGCCTGGAGTTCCTGCTGACCATCGGCGGCG ACACCCAGGCCGCCGCCCTGACCGGCGACAAGTACTTCGGCTTCCTGCTG GCCCTGCTGGCCATCTTCGGCGTGTCCTTCGAAGTGCCGCTGGTGATCGGC ATGCTGAACATCGTGGGCATCCTGCCGTACGACGCCATCAAGGACAAGCG CCGCATGATCATCATGATCCTGTTCGTGTTCGCCGCCTTCATGACCCCGGGC CAGGACCCGTTCACCATGCTGGTGCTGGCCCTGTCCCTGACCGTGCTAGTG GAACTGGCCCTGCAGTTCTGCCGCTTCAACGACAAGCGCCGCGACAAGAA GCGCCCGGAATGGCTGGACGGCGACGACCTGTCCGCCTCCCCGCTGGACA CCTCCGCCGGCGGCGAAGACGCCCCGTCCCCGGTGGAAACCCCGGAGGC CGTGGAACCGTCCCGCATGCTGAACCCGTCCGGCGAAGCCTCCATCTCCTA CAAGCCGGGCCGCGCCGACTTCGGCGACGTGCTGTGA |
| 21 | ATGCCTACTTTGGGTCCATGGGAGATCGCAATAATAGTCTTACTAATTATATT GTTATTTGGAGCAAAAAAACTTCCAGATGCAGCGAGATCTATAGGTCGTAG TATGCGAATATTTAAAAGTGAAGTCAAAGAGATGAATAAGGACGGTGATAC ACCTGAACAACAACAACAACAGCCACAACAACAACAACAAATCGCTCCA AATCAAATAGAAGCACCACAACCAGTTCAACAACCTGCGCAACAATCTAA TTTCGAACAACATTATCAGGGCCAACAAGTGCAGCAACCACAAAATCCTC AAACACCTGATTATAGACAAAATTATGAAGATCCAAATAGAACAAGTTGA |
| 22 | ATGTTTTCATCTGTTGGATGGGGAGAAATTTTTTTATTAGTTGTTGTGGGTT TGGTAGTGATTGGCCCAGAAAGACTTCCAAGATTAATACAAGATGCACGA GCTGCTTTGCTAGCAGCTCGTACAGCAATAGACAATGCAAAACAAAGTCTT GATAGTGATTTCGGATCAGAATTTGATGAAATTCGTAAACCTCTTACACAA GTTGCACAATACTCACGAATGAGTCCAAAAACTGCTATTACTAAAGCATTG |
| | TTTGATAATGATTCTTCATTTCTTGATGACTTTGACCCAAAAAAGATAATGG CTGAAGGTACTGAAGGTGAAGCACAAAGACACAAACAAGCTGCTGATAAT AATGCGAATGTGGTGGAAAGACCTGCAGATGGTTCTACGGCACGTCCAAC ACAAAATGATCCAAAAGATGGTCCAAATTACAGTGGTGGAGTTAGTTGGA CAGACATTATATAG |
| 23 | ATGAGTATCGTAGAACATATTAAGGAATTTCGTAGAAGATTGCTAATTGCAC TTGCAGGAATCTTAGTTGGTACTATTATTGGATTTATTTGGTACGATTTTTCA TTCTGGCAAATTCCTACTCTAGGTGAGTTATTACGTGATCCATATTGTAGTTT ACCAGCTGAGTCTCGTTGGGCAATGTCTGATTCTGAAGAATGTCGTTTACT AGCAACAGGTCCTTTTGATCCATTTATGTTACGACTTAAAGTAGCAGCACTT GTGGGAATGGTCTTAGGAAGTCCTGTTTGGTTATCTCAGTTGTGGGGTTTTA TAACGCCAGGCCTAATGAAAAATGAACGTCGTTATACTGCGATTTTCGTTA CAATTGCAGTTGTTTTGTTTGTAGGTGGCGCAGTCTTAGCATATTTTGTGGT AGCTTATGGTCTTGAGTTCTTACTTACTATTGGTGGTGACACGCAAGCTGC AGCATTAACAGGTGATAAATATTTTGGTTTTTTACTTGCATTATTAGCAATTT TTGGCGTGTCTTTCGAAGTGCCTTTAGTAATTGGTATGTTAAATATTGTGGG CATTTTACCTTATGATGCAATAAAAGACAAAAGACGTATGATCATTATGATT TTATTTGTTTTTGCTGCTTTCATGACGCCAGGTCAGGATCCATTTACTATGTT AGTATTAGCTTTAAGTTTAACAGTATTAGTAGAATTAGCATTACAATTCTGTC GTTTTAATGATAAACGTAGAGACAAAAAGCGTCCTGAATGGTTAGATGGAG ATGATCTTTCTGCATCACCTTTAGATACAAGTGCAGGCGGAGAAGATGCGC CATCACCTGTTGAAACACCTGAAGCTGTAGAACCTTCACGTATGCTTAATC CTAGTGGAGAAGCATCAATTTCATATAAACCAGGTCGAGCAGATTTTGGTG ACGTATTGTAG |
| 24 | MPTLGPWEIAIIVLLIILLFGAKKLPDAARSIGRSMRIFKSEVKEMNKDGDTPE QQQQQPQQQQQIAPNQIEAPQPVQQPAQQSNFEQHYQGQQVQQPQNPQTPD YRQNYEDPNRTS |
| 25 | MFSSVGWGEIFLLVVVGLVVIGPERLPRLIQDARAALLAARTAIDNAKQSLDS DFGSEFDEIRKPLTQVAQYSRMSPKTAITKALFDNDSSFLDDFDPKKIMAEGTE |
| | GEAQRHKQAADNNANVVERPADGSTARPTQNDPKDGPNYSGGVSWTDII |
| 26 | MSIVEHIKEFRRRLLIALAGILVGTIIGFIWYDFSFWQIPTLGELLRDPYCSLPAE SRWAMSDSEECRLLATGPFDPFMLRLKVAALVGMVLGSPVWLSQLWGFITPG LMKNERRYTAIFVTIAVVLFVGGAVLAYFVVAYGLEFLLTIGGDTQAAALTGD KYFGFLLALLAIFGVSFEVPLVIGMLNIVGILPYDAIKDKRRMIIMILFVFAAFM TPGQDPFTMLVLALSLTVLVELALQFCRFNDKRRDKKRPEWLDGDDLSASPL DTSAGGEDAPSPVETPEAVEPSRMLNPSGEASISYKPGRADFGDVL |
| 27 | ATGAATAATAATGATTTATTTCAAGCATCTAGACGACGATTTTTGGCTCAATT AGGCGGTTTGACAGTTGCTGGGATGTTGGGTCCTTCATTATTGACTCCTCG TCGTGCCACCGCA |
| 28 | ATGAACAACAACGACCTGTTCCAGGCCTCCCGCCGCCGCTTCCTGGCCCA GCTGGGCGGCCTGACCGTGGCCGGCATGCTGGGCCCGTCCCTGCTGACCC CGCGCCGCGCCACCGCC |
| 29 | ATGAACAATAATGACTTATTTCAAGCTTCACGTCGTTTCTTGGCACAAT TGGGTGGTTTGACAGTTGCAGGTATGCTTGGTCCAAGTTTGTTGACTCCAA GACGTGCAACTGCA |
| 30 | MANMFALILVIATLVTGILWCVDKFFFAPKRRERQAAAQAAAGDSLDKATLK KVAPKPGWLETGASVFPVLAIVLIVRSFIYEPFQIPSGSMMPTLLIGDFILVEKF AYGIKDPIYQKTLIETGHPKRGDIVVFKYPEDPKLDYIKRAVGLPGDKVTYDP VSKELTIQPGCSSGQACENALPVTYSNVEPSDFVQTFSRRNGGEATSGFFEVP KNETKENGIRLSERKETLGDVTHRILTVPIAQDQVGMYYQQPGQQLATWIVP PGQYFMMGDNRDNSADSRYWGFVPEANLVGRATAIWMSFDKQEGEWPTGL RLSRIGGIH |
| 31 | MKSENVSKKKSILEWAKAIVIAVVLALLIRNFIFAPYVVDGDSMYPTLHNRER VFVNMTVKYIGEFDRGDIVVLNGDDVHYVKRIIGLPGDTVEMKNDQLYING KKVDEPYLAANKKRAKQDGFDHLTDDFGPVKVPDNKYFVMGDNRRNSMDS RNGLGLFTKKQIAGTSKFVFYPFNEMRKTN |
| 32 | MTEEKNTNTEKTAKKKTNTYLEWGKAIVIAVLLALLIRHFLFEPYLVEGSSMY PTLHDGERLFVNKTVNYIGELKRGDIVIINGETSKIHYVKRLIGKPGETVQMK |
| | DDTLYINGKKVAEPYLSKNKKEAEKLGVSLTGDFGPVKVPKGKYFVMGDNR LNSMDSRNGLGLIAEDRIVGTSKFVFFPFNEMRQTK |
| 33 | MTDFSSASNADDSTQDGRPGRRAGKSKKESKPTPWYIEIPVVVVLTLALIFVL QTFVGRMYMIPSGSMEPTLHGCEGCTGDRILVEKVSYYFTDPEPGDVVVFKG TDSWNVGFTTQRSDNSVIRGLQNLGSYVGLVAPDENDLVKRIIATGGQTVSCQ AGDPGIMVDGKEVDDSYTLQPAQFPIDETSGSTECGGNYFGPITVPDGNYFM MGDNRTNSMDSRYHLGDQYQGTIPEENIKGKVQAIILPFSRIGGVDDPAIKG |
| 34 | MMKFLKEWGLFIFIIAAVLLSRVFIWSLVVVDGHSMDPTLADKERLVIVRTTKI NRFDIVVAKENAADGSTKDIVKRVVGMPGDTIKFDHDQLTINNKVYPENYLK DYQKQLADGQLEKTYGNYPLTKALTDQNRSLFVSLAQSTKAFTTDSTGNPTF TVKVPDGQYFLMGDNRVVSQDSRAVGSFKRSAIIGEAKLRVWPLNKISFF |
| 35 | ATGTTTTCAAACATTGGAATACCGGGCTTGATTCTCATCTTCGTCATCGCCC TCATTATTTTTGGCCCTTCCAAGCTGCCGGAAATCGGGCGTGCCGCCGGAC GGACACTGCTGGAATTTAAAAGCGCCACAAAATCACTTGTGTCTGGTGATG AAAAAGAAGAGAAATCAGCTGAGCTGACAGCGGTAAAGCAGGACAAAAA CGCGGGCTGA |
| 36 | ATGGATAAAAAAGAAACCCATCTGATCGGGCATTTAGAAGAGCTTCGCCGC CGGATTATCGTCACCCTTGCGGCATTTTTTCTATTTCTCATCACGGCTTTTTT GTTCGTACAGGACATTTATGACTGGCTGATCAGGGATTTGGATGGAAAGCT GGCTGTGCTAGGACCGAGTGAAATCCTCTGGGTGTATATGATGCTTTCCGG CATTTGTGCCATTGCGGCTTCTATCCCTGTTGCCGCGTACCAGCTGTGGCGT TTCGTTGCACCGGCGCTGACTAAAACGGAGCGCAAGGTGACGCTCATGTA CATACCAGGTTTATTTGCGTTGTTTTTGGCGGGCATCTCCTTCGGATACTTT GTCTTGTTTCCGATCGTGCTCAGCTTTTTGACTCATTTATCCTCCGGCCACT TTGAAACGATGTTTACGGCTGACCGCTACTTTAGGTTTATGGTGAATTTGAG CCTGCCGTTCGGCTTCTTGTTTGAGATGCCCTTGGTGGTGATGTTTTTAACA AGGCTGGGCATCTTAAATCCTTACAGACTGGCCAAAGCGAGAAAGCTTTC CTATTTTCTGCTGATTGTCGTGTCCATATTGATTACACCGCCTGATTTTATTT CTGATTTTCTCGTGATGATCCCGCTTCTTGTCCTGTTTGAAGTGAGTGTCAC |
| | CCTATCGGCGTTTGTCTACAAAAAGAGGATGAGGGAAGAAACAGCGGCGG CCGCTTAG |
| 37 | ATGCCGATCGGTCCTGGAAGCCTTGCTGTTATCGCAATCGTTGCTCTGATTA TCTTCGGTCCCAAAAAGCTGCCTGAATTGGGGAAAGCAGCGGGAGACAC ACTTCGTGAATTTAAAAACGCTACTAAAGGATTAACGAGTGATGAAGAGG AAAAAAAGAAAGAAGATCAGTAA |
| 38 | ATGACACGAATGAAAGTGAATCAAATGTCGCTGCTGGAGCATATTGCTGAG CTTCGAAAACGGTTGCTGATTGTAGCGCTGGCGTTTGTCGTTTTCTTTATTG CTGGATTTTTTTTAGCAAAGCCGATTATTGTGTATCTGCAAGAAACAGATGA AGCGAAGCAGCTTACGCTTAACGCGTTTAACCTGACAGACCCGCTTTATGT GTTTATGCAATTTGCGTTTATCATCGGCATAGTCTTGACCTCGCCGGTTATTC TTTATCAGCTTTGGGCTTTTGTGAGCCCGGGCCTCTATGAGAAAGAGCGCA AAGTAACGCTCAGCTACATTCCGGTCTCTATTTTGCTGTTTTTAGCGGGCTT ATCTTTTTCATATTATATTTTATTTCCTTTTGTTGTTGATTTTATGAAGCGGAT TTCTCAGGACTTGAATGTCAATCAGGTGATCGGAATTAATGAATATTTTCAT TTTCTTCTGCAGCTGACGATTCCGTTTGGACTGCTGTTCCAAATGCCGGTC ATCCTCATGTTTTTGACAAGGCTCGGAATTGTGACACCGATGTTCTTGGCG AAAATCAGAAAGTATGCGTATTTTACGCTGCTGGTGATCGCAGCCCTGATT ACACCGCCTGAGCTTCTGTCCCATATGATGGTCACAGTCCCGCTTTTGATTT TATATGAAATCAGTATCCTTATATCGAAGGCCGCTTATCGGAAAGCACAGAA AAGCAGTGCTGCCGATCGGGACGTGTCTTCTGGGCAATAA |
| 39 | MFSNIGIPGLILIFVIALIIFGPSKLPEIGRAAGRTLLEFKSATKSLVSGDEKEEKS AELTAVKQDKNAG |
| 40 | MDKKETHLIGHLEELRRRIIVTLAAFFLFLITAFLFVQDIYDWLIRDLDGKLAV LGPSEILWVYMMLSGICAIAASIPVAAYQLWRFVAPALTKTERKVTLMYIPGLF ALFLAGISFGYFVLFPIVLSFLTHLSSGHFETMFTADRYFRFMVNLSLPFGFLFE MPLVVMFLTRLGILNPYRLAKARKLSYFLLIVVSILITPPDFISDFLVMIPLLVLF EVSVTLSAFVYKKRMREETAA |
| 41 | MPIGPGSLAVIAIVALIIFGPKKLPELGKAAGDTLREFKNATKGLTSDEEEKKK |
| | EDQ |
| 42 | MTRMKVNQMSLLEHIAELRKRLLIVALAFVVFFIAGFFLAKPIIVYLQETDEA KQLTLNAFNLTDPLYVFMQFAFIIGIVLTSPVILYQLWAFVSPGLYEKERKVTLS YIPVSILLFLAGLSFSYYILFPFVVDFMKRISQDLNVNQVIGINEYFHFLLQLTIP FGLLFQMPVILMFLTRLGIVTPMFLAKIRKYAYFTLLVIAALITPPELLSHMMV TVPLLILYEISILISKAAYRKAQKSSAADRDVSSGQ |
| 43 | ATGCCTAATCTTCGCCCCAGCGAGCTCATTATCATTCTGCTTATCATCGTGCT GCTGTTTGGCGCGAAGAAGCTCCCTGAGCTAGCCAAAAGCATTGGCCAAT CGATGAAGATTTTCCGTGCCGAGACCAGGACCGATGACCCGGCAACACAA CAAACCGTCAATACAGTCAGCGACGCCCCTGCCCCAGCTCCGGCGGCAGC ACCGGCTCCCGCTCCCGCCGTACCCCAAGCCGTCACGATGAACGCCACCG CCGCCCCGACTACAGCAACCGGAGCCGAACAGCGATGA |
| 44 | ATGTTGTTCGGCATTTCGGGCACGGAACTCATGGTGATTCTTGTCATCGCTT TAGTGCTGGTAGGCCCCGACAAGCTGCCCGACTACGCACGCAAGGTCGGA CGATTTATCCATAGCATGCGCGTACGCGGCCAAGCGTTGAGCGAACAAAGC AACATCGACGTTCACGGCCTTGTACAGGATTCCGGCATCAATGATATTCGC AAAGGTCTGGATGATGCCACGCAAGATGTGAACCGGCTGCTGCCGCCTAT CCGGTGA |
| 45 | ATGAGCACGGTTCCCGGAAACCCTGCGCTTCCGGCACCCCTTGCACCGGC GATGCATCCCGGCGATCGGGATACACATCTTGACCTCGACGATCTCATCGAT CACTCACACGAGGCCACGCAGCAGGCCAGCGCGGGTGCGCAACACCCTA CACGCCGGTGGAAGCGGGGTGTAGGGTGCAAGTCCAAAACGCGCAAGCC CCGCAACCCCGATGCGGTGATGCCTCTGGCCGATCATCTTGTCGAATTTCG CAAACGCTTTGTTCGGGCCATTGCCGGCATCATCATCATGTCGATAGTGGG ATGGATGTTCAGCGATCAGGTGTTTCGTATCCTGCAACAACCGTTCCTGAC GGCGGCCGGGCAGCAACAGGGACTGATGAGCATCACATTCAATGGCGTGG TCTCCGCATTCAACGTAAAGCTGGAGATTGCGTTCTTCCTTGGTCTGACGG CCTCCTGCCCGTGGTGGTCATACCAAGTGTGGGCATTCATTAATCCAGGGT TGAAGCGCAAAGAGCGCTGGACCGCGGTTACATTCATCGGAGCCTCAGTG |
| | CCGCTGTTCCTCACCGGAGCCGGACTGGCATGGTATCTGCTGCCCCAAGCG GTGGCGATCCTTACCGGCTTTGCCCCCGCAAACACCGCCACATTGCTCAGC GCCGACGTCTATTTTGATTTCATTCTGCGCATGACAGTCGCTTTCGGCCTCT CATTCCTGCTACCCGTAGTTATGGCGGCATTGACGATGATGAATGCGGTCG AAACACGAACATGGTTGAAGCAGTGGCGTCTCGCAACGGTCATAGCGTTT ATCTTCGCGGCCGTGGCCACGCCAACCGGCGACCCAGGAACATTATGCGC ACTCGCTTTGCCCATCATCGCAATCTATTTCGCAGCCATAGCCGTATGCGCA GTATACGAGCACATCCAGCTATGGAAAGTCATGCGCGCGGCGGGCGAAGA ACCCAAACTTCTCAAACTTTTACATAAAATGCGCGCCGCGTTACCATTTCG CGCGGCGCGCAAAGCCAAGCGCACCGGCAAACAGGAAGCCCCTGCAACA ATAACTGCAGAAGGAGAATAA |
| 46 | MPNLRPSELIIILLIIVLLFGAKKLPELAKSIGQSMKIFRAETRTDDPATQQTVNT VSDAPAPAPAAAPAPAPAVPQAVTMNATAAPTTATGAEQR |
| 47 | MLFGISGTELMVILVIALVLVGPDKLPDYARKVGRFIHSMRVRGQALSEQSNI DVHGLVQDSGINDIRKGLDDATQDVNRLLPPIR |
| 48 | MSTVPGNPALPAPLAPAMHPGDRDTHLDLDDLIDHSHEATQQASAGAQHPTR RWKRGVGCKSKTRKPRNPDAVMPLADHLVEFRKRFVRAIAGIIIMSIVGWMF SDQVFRILQQPFLTAAGQQQGLMSITFNGVVSAFNVKLEIAFFLGLTASCPWW SYQVWAFINPGLKRKERWTAVTFIGASVPLFLTGAGLAWYLLPQAVAILTGFAP ANTATLLSADVYFDFILRMTVAFGLSFLLPVVMAALTMMNAVETRTWLKQW RLATVIAFIFAAVATPTGDPGTLCALALPIIAIYFAAIAVCAVYEHIQLWKVMRA AGEEPKLLKLLHKMRAALPFRAARKAKRTGKQEAPATITAEGE |
| 49 | MNNNDLFQASRRRFLAQLGGLTVAGMLGPSLLTPRRATA |
| 50 | MSLSRRQFIQASGIALCAGAVPLKASA |
| 51 | MAYDSRFDEWVQKLKEESFQNNTFDRRKFIQGAGKIAGLSLGLTIAQS |
| 52 | MQQDGTQQDRIKQSPAPLNGMSRRGFLGGAGTLALATASGLLLPGTAHA |
| 53 | MMNLSRRTLLTTGSAATLAYALGMAGSAQA |
| 54 | TAATTCCTCCTTTTTGTTGACATTATATCATTGATAGAGTTATTTGTCAAACT AGTTTTTTATTTGGATCCCCTCGAGTTCATGAAAAACTAAAAAAAATATTGA |
| | CACTCTATCATTGATAGAGTATAATTAAAATAAGCTTG |
| 55 | GTGGGTGACAATTGATTTTGATAGTCCTGATATTACTTATGATGAATACTACG GTTATAAGGTCGTGGCTAATACTCATGCAGTAATATTAGATGGCTACAATGA AGCGACAAAAGAGTTTCATGTAACTGACCCAATCAAAGGGAAATACTGGT TAGCTGAATCTACGGTTGCCTCAGTTTACAATGAAACAAATCAATTTGCGA TTGAATTTTTAAAATAAAATTAGCACTCTGATAAAAAGAGTGCTAATTTTTT TGCCAAAAGTGGAAAAACGTGGTAAAATAGTGCTATTGAAAAATTGATTTA GTAAAGGAAGTGAGTAATTTAGAAA |
| 56 | ATTTTACTACTTAAGAGATAAAAAAGCTAGATATATGAAAAAACTTATTGAA AAAATGCGATTAAAGGCTGATAAGACTTGCATTTCAAAACGATTTTAAGTAT AATGATAAGGAACGGTAGATTGCAATTATGCAGACTACCTATAATAAAACA AATTCCCCAAAGGAGACATTTTTAAA |
| 57 | AAAACGTTTCATACAGTTTGTAAAGAGATTTTTTTATAAATACGTGATATAAT GAACTAGACAACAAATGAACATGGTAAAAATATTAATAACGTTTACGTATTT TTGCTGTGTATCTCAATTTCGGAGGACAGATTTAA |
| 58 | CTTTTGCTCAATATTATCTTAGCTGTAGCCTTACAATTCCCTTTAGAAATCTT TTACAGATTAAAGAAAAGTCATGTAAGATACAATTAGAAAGTGTTTTGTAA TCATAAAGAAATATTAAGGTGGGGTAGGAATAGTATAATATGTTTATTCAAC CGAACTTAATGGGAGGAAAAATTAAAAAAGAACAGTT |
| 59 | CGCATATCGCGGCGCGGCGCATCTCAATGCGTCGCGCCGCACGTTAATCTG CTGGCGAAACCCAGAGTTCTCCCAGCGGCCGATGGGTATCATGAACGTGC AAGCCAACCAAAGCGATGGAAGGGAGTCCGTT |
| 60 | CTTGCATGCCGCGCGCTTGCTCATGATGATTCGAGACATTCCTCCAAAGGA GAGGAAAAATCACATGTCGGTTGCGGGCAATCCGAATTGTGAGCGCTCAC AAAAAATACGGCATTTTTGCCCAAAACGCACGCTGAAACGTTGCCATGTGT ACAGAGTCGGCATTACAGTAGCAACTGTTGGTAAACAATGGCCCGGTGTG CCAAAGCGCGCCAAGGCCACCCTACAAGGGAGAATTAC |
| 61 | CACGCGCCACTGCATGAAGTAGCCAGTGTCTTCAGTCAGCGGTAATATCGT ATACCGCTGACTGGGTTCTGGCTCTTCAAAGTGGCACATAACCCAGAAACC |
| | CAGTAGAATAAGCGAGTTCCTGTGCGAAAGGGTGCAGGAAACTCACGAG ACGTCCAGGAGGACAAAAGTA |
| 62 | AGATCTAGTCTTATAACTATACTGACAATAGAAACATTAACAAATCTAAAAC AGTCTTAATTCTATCTTGAGAAAGTATTGGTAATAATATTATTGTCGATAACG CGAGCATAATAAACGGCTCTGATTAAATTCTGAAGTTTGTTAGATACAATGA TTTCGTTCGAAGGAACTACAAAATAAATTATAAGGAGGCACTCACCATGGG TACTGCAGGCATGCGGTACCACTAGTTCTAGAGAGCTCAAGCTTTCTTTGA ACCAAAATTAGAAAACCAAGGCTTGAAACGTTCAATTGAAATGGCAATTA AACAAATTACAGCACGTGTTGCTTTGATTGATAGCCAAAAAGCAGCAGTTG ATAAAGCAATTACTGATATTGCTGAAAAATTGTAATTTATAAATAAAAATCA CCTTTTAGAGGTGGTTTTTTTATTTATAAATTATTCGTTTGATTTCGCTTTCGA TAGAACAATCAAATCGTTTCTGAGACGTTTTAGCGTTTATTTCGTTTAGTTA TCGGCATAATCGTTAAAACAGGCGTTATCGTAGCGTAAAAGCCCTTGAGCG TAGCGTGGCTTTGCAGCGAAGATGTTGTCTGTTAGATTATGAAAGCCGATG ACTGAATGAAATAATAAGCGCAGCGTCCTTCTATTTCGGTTGGAGGAGGCT CAAGGGAGTTTGAGGGAATGAAATTCCCTCATGGGTTTGATTTTAAAAATT GCTTGCAATTTTGCCGAGCGGTAGCGCTGGAAAATTTTTGAAAAAAATTTG GAATTTGGAAAAAAATGGGGGGAAAGGAAGCGAATTTTGCTTCCGTACTA CGACCCCCCATTAAGTGCCGAGTGCCAATTTTTGTGCCAAAAACGCTCTAT CCCAACTGGCTCAAGGGTTTGAGGGGTTTTTCAATCGCCAACGAATCGCC AACGTTTTCGCCAACGTTTTTTATAAATCTATATTTAAGTAGCTTTATTTTTG TTTTTATGATTACAAAGTGATACACTAATTTTATAAAATTATTTGATTGGAGT TTTTTAAATGGTGATTTCAGAATCGAAAAAAAGAGTTATGATTTCTCTGAC AAAAGAGCAAGATAAAAAATTAACAGATATGGCGAAACAAAAAGATTTTT CAAAATCTGCGGTTGCGGCGTTAGCTATAGAAGAATATGCAAGAAAGGAAT CAGAACAAAAAAAATAAGCGAAAGCTCGCGTTTTTAGAAGGATACGAGTT TTCGCTACTTGTTTTTGATAAGGTAATTATATCATGGCTATTAAAAATACTAA AGCTAGAAATTTTGGATTTTTATTATATCCTGACTCAATTCCTAATGATTGGA AAGAAAAATTAGAGAGTTTGGGCGTATCTATGGCTGTCAGTCCTTTACACG |
| | ATATGGACGAAAAAAAAGATAAAGATACATGGAATAGTAGTGATGTTATAC GAAATGGAAAGCACTATAAAAAACCACACTATCACGTTATATATATTGCACG AAATCCTGTAACAATAGAAAGCGTTAGGAACAAGATTAAGCGAAAATTGG GGAATAGTTCAGTTGCTCATGTTGAGATACTTGATTATATCAAAGGTTCATA TGAATATTTGACTCATGAATCAAAGGACGCTATTGCTAAGAATAAACATATA TACGACAAAAAAGATATTTTGAACATTAATGATTTTGATATTGACCGCTATAT AACACTTGATGAAAGCCAAAAAAGAGAATTGAAGAATTTACTTTTAGATAT AGTGGATGACTATAATTTGGTAAATACAAAAGATTTAATGGCTTTTATTCGC CTTAGGGGAGCGGAGTTTGGAATTTTAAATACGAATGATGTAAAAGATATT GTTTCAACAAACTCTAGCGCCTTTAGATTATGGTTTGAGGGCAATTATCAGT GTGGATATAGAGCAAGTTATGCAAAGGTTCTTGATGCTGAAACGGGGGAA ATAAAATGACAAACAAAGAAAAAGAGTTATTTGCTGAAAATGAGGAATTA AAAAAAGAAATTAAGGACTTAAAAGAGCGTATTGAAAGATACAGAGAAAT GGAAGTTGAATTAAGTACAACAATAGATTTATTGAGAGGAGGGATTATTGA ATAAATAAAAGCCCCCCTGACGAAAGTCGACGGCAATAGTTACCCTTATTA TCAAGATAAGAAAGAAAAGGATTTTTCGCTACGCTCAAATCCTTTAAAAAA ACACAAAAGACCACATTTTTTAATGTGGTCTTTTATTCTTCAACTAAAGCAC CCATTAGTTCAACAAACGAAAATTGGATAAAGTGGGATATTTTTAAAATATA TATTTATGTTACAGTAATATTGACTTTTAAAAAAGGATTGATTCTAATGAAG AAAGCAGACAAGTAAGCCTCCTAAATTCACTTTAGATAAAAATTTAGGAGG CATATCAAATGAACTTTAATAAAATTGATTTAGACAATTGGAAGAGAAAAG AGATATTTAATCATTATTTGAACCAACAAACGACTTTTAGTATAACCACAGA AATTGATATTAGTGTTTTATACCGAAACATAAAACAAGAAGGATATAAATTT TACCCTGCATTTATTTTCTTAGTGACAAGGGTGATAAACTCAAATACAGCTT TTAGAACTGGTTACAATAGCGACGGAGAGTTAGGTTATTGGGATAAGTTAG AGCCACTTTATACAATTTTTGATGGTGTATCTAAAACATTCTCTGGTATTTGG ACTCCTGTAAAGAATGACTTCAAAGAGTTTTATGATTTATACCTTTCTGATG TAGAGAAATATAATGGTTCGGGGAAATTGTTTCCCAAAACACCTATACCTG AAAATGCTTTTTCTCTTTCTATTATTCCTTGGACTTCATTTACTGGGTTTAAC |
| | TTAAATATCAATAATAATAGTAATTACCTTCTACCCATTATTACAGCAGGAAA ATTCATTAATAAAGGTAATTCAATATATTTACCGCTATCTTTACAGGTACATC ATTCTGTTTGTGATGGTTATCATGCTGGATTGTTTATGAACTCTATTCAGGAA TTGTCAGATAGGCCTAATGACTGGCTTTTATAATATGAGATAATGCCGACTG TACTTTTTACAGTCGGTTTTCTAATGTCACTAACCTGCCCCGTTAGTTGAAG AAGGTTTTTATATTACAGCTCC |
| 63 | GGATCCTCATCAGCACCACCCGCGACACGAACGTATTACATACGCAATCAT ATCAGTGTCACACATCAGGTACAACCGTACCGCAACACGCTTCGAGCGGA CGCAAGCGGCTTTTGCGCGGAGCATTTCCGGCCATTTGCCCAGCTTGTTCC GCGCTTCCGAGAAGGTGTGGAGTTGGGTTTTCTGTGATGCCGCGCGAGCG GTCTGGAGGCTCCGCCGACCATGGGGCATGGGTCTTCCCGGCAAGCCGAT TCGCACCGCGAATCGAGTGATTGTAGCTACAATCACGTCGCTTGCTACTCC GGCCTTCGGCACTCGGGTTGTTGCTCCTGCGCCGAAGGGCGAGGGAGGCT GCGGGCTCGGTTCATGTACATGAACCGTCTAGCTTGCTTACCTTCGATTTGA TGGACATCTCCATGTGGATGTCCATGGAGATGTCCATGGAGATGTCCATGG AGATGTCCATGGAGATGTCCATGGAGATGTCCATGTGGGTGTCCATGTGGG TGTCCATGGATATATCCATCTGAGCGCATAGGAAAACGCATGAGGGTGGAT GATCGTTCGGGGTGATGCCTTCTTCCACGAACGGGAAGGGGCTCTTCCTTT TTCTGGAATGTCATCTTCCAGCGCCCTTCGCCTTGAGCTCCTTGAGCAGCA TCGTCGCGTTGTCGCGGATGCTCGCGTCCGTGAGCGGGGAGAGCTCGTCC GCATAGGAGCGGCAGAGGAGCGCGTACACCCGTTCCCGCACCCAGGCGCT GCGCGCGATGCCCAGCTTGAACGCGGCCCGGCACAGCATCTCGTATTCGTC GCCGGTGAGCAGGACATTGAGCTGGCGTCAAAAGTTGATTAGTCATGCCG TGTATATACGTCCGGGGTGTTTTCGGCCAGATTCCAACAGTTTTGGGCCAC TCCGGGAAGGAACAGCCGACGCTTCGCTGGGCTGAACCATCTGACTTGGT TCGGCGCACGCTTGTGCAATGCACAAATGTGCAATAGGATGTGAGCTATGA GCAACGAATATGTGCAGGGGACGCTGGAGCTGACGCGGGCGTTCGACGGC TGGTGGCTTCCCCAGCGTCCCCTGTGCTGCGACGATGACTACTCCCAGCTG GTGCGCCGGAGCCGCATCGACGCGCTCAGATGCAAGCACATCGAGGCGAA |
| | TCCGGCAGCGCAGGTGAACATGCTCGTTGTTGACGTTGATTCGTCTGAGG GCCGTTTAATGAGCCTGTGGGGGCATCGGGACATGCCGCCCAACTTCATCG CGGAGAATCCGGCCAACGGCCACTGCCACGCGGGCTGGGTGCTCACCGA ACCCGTCTGCCGCACCGACATGGCCAGACTCAAGCCCCTGAAGCTCCTGC ACGCCGTCACCGAGGGGCTGCGCCGCAGCGTGGACGGCGACGAGGGCTA TTCCGGCCTGCTCATGAAGAACCCGTTGAGCGACGCGTGGGACAGCGACC TGTGCCGCGAGGACACCTACGACCTGCCCGACCTCGTGGCCGCGCTCGAA GCGCACGGGGACATGCCGCCCAAGAGCTGGACGCGCACCAAACGCGCCC GCGAGGTCGGAGTGGGCCGCAACTGCACCCTGTTCGACGAGGCCCGCAC CCTCGCCTACCGCGAGGTGCGCCGACTGCCTGACCGCACGCCCGCCTCCT CCGACCTGCTGCGCGAGTACGTGCGCCGCACCTGCCACGAAATCAACGCC TCGTTCCCCGACCCGCTGCCCGTGCGCGAGGTCAACGACACCGCCAAGAG CATCCACAAGTGGATCACCACGCGAAGCCGCATGTGGCGAGACGGTGCCG TCGCCAACGCCGCCACGTTCGTCGCCATCCAATCCGCACGCGGAAAGAAA TCGGGCGAGGCCAGACAAAACGCATTTGAAGAGAAGTTCGCGCAGTACG CGCAGGAGGTGCTTGGACAATGACCATCCAGACAATTCGCAAGAAGCGCC CATTGCCCGCCAAGGAGTTAGCCGAGGCCTATGGGGTGTCCGTGCGCACG ATCAAGTACTGGAATTCGCAAACGCGCGAGGACTGGATAGACGAGCAGGC AACGTTGCGCGAATCCATCCGCGCCTACCACGACGATGACGGCCACTCGT GGAGCCAGACCGCCGAACACTTCAACATGACCCAAGGTGCCGTGCGCCA ACGCGCCTACCGCGCCCGCAAGGAACGCGAGGCCGAGGCAAAAGCCGCA AGGCCGGAGTAGCAAGTGACGTGAACGTATATACGTTCACTCGATTCGCGG TGCGAATCGGCTTGCCGGGAAGACCCATGCCCCATAGTCGGCAGAGCCTC CAGACCGCTCACGCGGCATCGTGCCACGACTGTGGCACATCACGTAACAC CCGCACATCCTCACGGTGGTGCGCGGGTGTTACGTGATTCGAACCATCGGC CTGACAGGTCTCAGATCGCTCCAGCGAGCTTACGGCGCATGTACTCGCTGC GGCTGATGTGGTACCGCCTGGCCATCGCGGTCAGACCGTCCAGCGTGCTCT TGGGCATGCGGACGGACACGCTGACCATCTGCTCGTCGGGCTCGGCGAGA TGGAGCCCGTAGTACACGCGCCCCGTCAGGTCGTCCGGGACAGTCTCGGA |
| | CTCGGCCATCCGCTCATCCTCCAGCACCTGCTCCTCGGTCAGCCCGACCAG CGCCATCAACTGCCTGTCGCGTTCGCTGAAATCACTCATAGCGTCCTCCCA TCGCCGCGCAGACGGTCTATCTCGCGCCTGAACTTCTTGGTCGGCGGCGTG AACGCGTGGTAGATCACCACGAGGTCGCCGACGACCCTGTACAGCATCTC GACGTTCCTGCCGCGCCCGTCGAGCCCTATCGCCATCCACGCGCCGGACTC GCGCTCCGCGTCCACCATGACGCTGCGGAACGCGGTCACCACGTCCTTCT CGGACAGTTCTGGGTGACGCTCCATCACCCTGTCAAGCACCTGGATCCCC GGGTACCGAGCTCGAATTCTGAGCAAAAGGCCAGCAAAAGGCCAGGGAC CGTAAAAAGGCCGCGTTGCTGGCGTTTTTCCATAGGCTCCGCCCCCCTGAC GAGCATCACAAAAATCGACGCTCAAGTCAGAGGTGGCGAAACCCGACAG GACTATAAAGATACCAGGCGTTTCCCCCTGGAAGCTCCCTCGTGCGCTCTC CTGTTCCGACCCTGCCGCTTACCGGATACCTGTCCGCCTTTCTCCCTTCGGG AAGCGTGGCGCTTTCTCATAGCTCACGCTGTAGGTATCTCAGTTCGGTGTA GGTCGTTCGCTCCAAGCTGGGCTGTGTGCACGAACCCCCCGTTCAGCCCG ACCGCTGCGCCTTATCCGGTAACTATCGTCTTGAGTCCAACCCGGTAAGAC ACGACTTATCGCCACTGGCAGCAGCCACTGGTAACAGGATTAGCAGAGCG AGGTATGTAGGCGGTGCTACAGAGTTCTTGAAGTGGTGGCCTAACTACGGC TACACTAGAAGAACAGTATTTGGTATCTGCGCTCTGCTGAAGCCAGTTACC TTCGGAAAAAGAGTTGGTAGCTCTTGATCCGGCAAACAAACCACCGCTGG TAGCGGTGGTTTTTTTGTTTGCAAGCAGCAGATTACGCGCAGAAAAAAAG GATCTCAAGAAGATCCTTTGATCTTTTCTACGGGGTCTGACGCTCAGTGGA ACGAAAACTCACGTTAAGGGATTTTGGCCATGAGATTATCAAAAAGGATCT TAACCTAGATCCTTTTAAATTAAAAATGAAGTTTTAAATCAATCTAAAGTATA TATGAGTACTGAATTCCCATTAAATAATAAAACAAAAAAATTAAAAAAGTG TTTCCACCATTTTTTCAATTTTTTTATAATTTTTTTAATCTGTTATTTAAATAG TTTATAGTTAAATTTACATTTTCATTAGTCCATTCAATATTCTCTCCAAGATAA CTACGAACTGCTAACAAAATTCTCTCCCTATGTTCTAATGGAGAAGACTCA GCCACTGCATTTCCCGCAATATCTTTTGGTATGATTTTACCCGTGTCCATAGT TAAAATCATACGGCATAAAGTTAATATAGAGTTGGTTTCATCATCCTGATAAT |
| | TATCTATTAATTCCTCTGACGAATCCATAATGGCTCTTCTCACATCAGAAAAT GGAATATCAGGTAGTAATTCCTCTAAGTCATAATTTCCGTATATTCTTTTATTT TTTCGTTTTGCTTGGTAAAGCATTATGGTTAAATCTGAATTTAATTCCTTCTG AGGAATGTATCCTTGTTCATAAAGCTCTTGTAACCATTCTCCATAAATAAAT TCTTGTTTGGGAGGATGATTCCACGGTACCATTTCTTGCTGAATAATAATTG TTAATTCAATATATCGTAAGTTGCTTTTATCTCCTATTTTTTTTGAAATAGGTC TAATTTTTTGTATAAGTATTTCTTTACTTTGATCTGTCAATGGTTCAGATACG ACGACTAAAAAGTCAAGATCACTATTTGGTTTTAGTCCACTCTCAACTCCT GATCCAAACATGTAAGTACCAATAAGGTTATTTTTTAAATGTTTCCGAAGTA TTTTTTTCACTTTATTAATTTGTTCGTATGTATTCAAATATATCCTCCTCACTA TTTTGATTAGTACCTATTTTATATCCATAGTTGTTAATTAAATAAACTTAATTT AGTTTATTTATAGATTTCATTGGCTTCTAAATTTTTTATCTAGATAATAATTAT TTTAGTTAATTTTATTCTAGATTATATATGATATGATCTTTCATTTCCATAAAA CTAAAGTAAGTGTAAACCTATTCATTGTTTTAAAAATATCTCTTGCCAGTCA CGTTACGTTATTAGTTATAGTTATTATAACATGTATTCACGAACGAAAATCGA CCTGCAGGCATGCAAGCTTGCATGCCTGCAGGATAGGCAGGCCAGCTCAA G |
| 64 | ATGACAGATTTTTCATCAGCTTCAAATGCTGATGATTCAACACAAGATGGA CGTCCAGGACGTCGTGCTGGAAAATCAAAAAAAGAATCAAAACCAACAC CATGGTATATTGAAATTCCAGTTGTTGTTGTTTTAACATTAGCTCTTATTTTC GTTTTACAAACATTTGTCGGACGTATGTATATGATTCCATCAGGATCAATGG AACCAACATTACATGGATGTGAAGGATGTACAGGAGATCGTATTTTAGTTG AAAAAGTTTCATATTATTTTACAGATCCAGAACCAGGAGATGTTGTTGTATT CAAAGGAACAGATTCATGGAATGTTGGATTCACAACACAACGTTCAGATAA TTCAGTTATTCGTGGATTACAAAATCTTGGATCATATGTTGGATTAGTTGCTC CAGATGAAAATGATCTTGTTAAACGTATTATTGCTACAGGTGGACAAACAG TTTCATGTCAAGCTGGAGATCCAGGAATTATGGTTGATGGAAAAGAAGTTG ATGATTCATATACATTACAACCAGCTCAATTTCCAATTGATGAAACATCAGG ATCTACAGAATGTGGAGGAAATTATTTTGGACCAATTACAGTTCCAGATGG |
| | AAATTATTTCATGATGGGAGATAATCGTACAAATTCAATGGATTCACGTTAT CATCTTGGAGATCAATATCAAGGAACAATTCCAGAAGAAAATATTAAAGGA AAAGTTCAAGCTATTATTCTTCCATTTTCACGTATCGGAGGAGTTGATGATC CAGCTATTAAAGGATAA |
| 65 | CAATGTCAGCGAAAAGTTAAAGGAGAAGGCATGACAGATTTTTCATCAGC TTCAA |
| 66 | TTTTGGTTCAAAGAAAGCTTTTATCCTTTAATAGCTGGATCATCA |
| 67 | AAGCTTTCTTTGAACCAAAATTAGA |
| 68 | TTAACTTTTCGCTGACATTGGAAG |
| 69 | CAATGTCAGCGAAAAGTTAAAGGAGAAGGCATGATGAAATTTTTAAAAGA ATGGG |
| 70 | TTTTGGTTCAAAGAAAGCTTTTAAAAGAAAGAAATTTTATTGAGT |
| 71 | TACATTTACAAATGAACAGATAGTTGATATTATATTTAAGAATTCTTCTTCAG AGCCTAAGATTAAAGCTTTCAATTGGCGAAAAGAAGTTGTACAATATGTAT AAGGGTATGTCAGTCACCGAATCAGATGATCTGGCATTATACTTGTAAATTA TCAGGAGGTTTTCAT |
| 72 | METNVSRRDFLKGSIVVALGAAGAATLSSCAGGDAAAKADSAVA |
| 73 | ATGGAAACGAACGTCTCGCGTCGTGATTTTCTTAAGGGAAGCATCGTGGTC GCATTGGGCGCGGCCGGTGCTGCTACGCTCAGCTCGTGCGCAGGCGGCGA TGCTGCAGCGAAAGCTGATTCTGCAGTTGCA |
| 74 | MKAFREAMSWIIPILIGLIIALVIKQFFFQIVRVDGPSMQPNLQNNERVFCLKTA KIHHGSVVVFDANGVDPQVSVKTDYVKRVIGLPGDTVSSRNGNIYVNGKKIN QDYISKSQRTTGTGNWTLRSISVQNSWLKNNGATKVPKGEYFVLGDHRSVSN DGRYWGFVPKSKIDGVVKVPSWTGTKTTRENVNKEWQHFYDK |
| 75 | MVGRHSKKARGQSRRYARKPSVWSEIRGYLLTVIAVMLVVLLGRTFVFNVYV IPSRSMEDTLQIGDRVFASRLTPRLFTLHRGDIIVFKDPADWMEGEQLPTNLMS IIDSNRYLIKRVIGLPGDTVACKGSGEPITVNGKPIDESAYLKSGVNPSDSPFSV |
| | TVTDGNVFVLGDNRSNSRDSRYHLDDGNNGLVPYDDIQGVALFRFWPFTRIG ILN |
| 76 | ATGAAAGCATTTCGTGAAGCAATGAGTTGGATTATTCCCATTCTAATCGGGT TAATAATCGCATTAGTAATTAAGCAGTTTTTCTTCCAAATTGTTCGGGTTGAT GGACCATCCATGCAACCAAACTTACAAAATAATGAACGAGTATTTTGTTTG AAAACTGCCAAAATCCATCATGGAAGTGTCGTTGTATTTGATGCTAATGGC GTTGATCCGCAAGTATCAGTTAAAACCGATTATGTTAAGCGAGTAATTGGA CTTCCTGGTGATACTGTTAGCTCTAGGAATGGAAATATTTATGTTAACGGCA AAAAGATTAATCAAGACTACATTAGCAAAAGTCAACGAACAACCGGAACT GGGAACTGGACACTGCGTAGTATCTCAGTTCAGAACAGTTGGTTAAAGAAT AATGGTGCCACAAAAGTTCCGAAAGGTGAATACTTTGTTCTTGGGGACCA CCGGAGCGTTTCAAATGATGGTCGTTACTGGGGATTTGTACCTAAGAGTAA GATTGATGGGGTTGTAAAAGTTCCATCATGGACTGGGACAAAAACGACTC GCGAAAACGTTAATAAAGAATGGCAGCACTTCTATGATAAGTAA |
| 77 | ATGGTGGGGCGTCATAGCAAAAAAGCGCGTGGCCAGTCGAGGAGATATGC GCGAAAGCCCTCCGTTTGGAGCGAAATTCGCGGCTATCTGCTGACAGTGAT CGCGGTGATGCTGGTCGTGTTGCTGGGACGTACCTTCGTGTTCAACGTATA TGTGATTCCATCCCGCAGCATGGAAGACACGTTGCAAATCGGCGACCGCGT ATTTGCCTCGCGATTGACTCCGAGACTCTTTACACTGCACCGTGGCGACAT CATCGTGTTCAAAGATCCCGCAGACTGGATGGAGGGGGAGCAGCTTCCCA CCAATCTGATGTCGATAATCGACAGCAATCGTTACCTAATCAAACGTGTCAT CGGTCTGCCCGGCGATACCGTCGCCTGCAAGGGATCCGGGGAGCCCATCA CGGTCAACGGCAAACCCATTGATGAAAGCGCTTATCTTAAGTCCGGCGTGA ATCCAAGCGACAGCCCTTTTTCCGTAACCGTTACTGACGGCAACGTGTTCG TGCTTGGTGATAATCGCTCCAATTCCAGAGATTCTCGATACCATCTTGACGA CGGCAACAATGGGCTAGTGCCATACGACGATATCCAAGGTGTGGCGTTGTT CCGCTTCTGGCCATTTACGAGAATCGGCATACTGAACTGA |
| 78 | MKKEILEWIVAIAVAIALIAIITKFVGKSYSIKGDSMDPTLKDGERVVVNIIGYK LGGVEKGNVIVFHANKKDDYVKRVIGTPGDSVEYKNDTLYVNGKKQSEPYL |
| | NYNEKRKQTEYITGSFKTKNLPNANPQSNVIPKGKYLVLGDNREVSKDSRSF GLIDKDQIVGKVSLRYWPFSEFKSNFNPNNTKN |
| 79 | ATGGATCCTACATTAAAAGATGGGGAGCGTGTAGTGGTAAATATTATTGGCT ATAAATTAGGTGGCGTTGAAAAAGGAAATGTCATTGTATTTCATGCTAATAA AAAAGATGATTATGTTAAAAGAGTTATTGGAACTCCAGGAGATAGTGTTGA ATATAAAAATGATACACTCTATGTTAATGGTAAAAAGCAATCAGAACCATAC TTGAACTATAATGAAAAACGTAAGCAAACTGAGTATATCACAGGTAGTTTC AAAACAAAAAATTTACCAAATGCTAATCCTCAATCTAATGTTATTCCTAAAG GTAAATATTTAGTTTTGGGGGATAACCGTGAGGTAAGTAAAGATAGTCGTT CATTCGGTTTAATTGACAAAGACCAAATTGTTGGAAAGGTATCGCTCAGAT ATTGGCCTTTCAGTGAATTTAAATCTAACTTTAATCCAAATAACACTAAAAA TTAA |
| 80 | ATGGCGAATATGTTTGCCCTGATTCTGGTGATTGCCACACTGGTGACGGGC ATTTTATGGTGCGTGGATAAATTCTTTTTCGCACCTAAACGGCGGGAACGT CAGGCAGCGGCGCAGGCGGCTGCCGGGGACTCACTGGATAAAGCAACGT TGAAAAAGGTTGCGCCGAAGCCTGGCTGGCTGGAAACCGGTGCTTCTGTT TTTCCGGTACTGGCTATCGTATTGATTGTGCGTTCGTTTATTTATGAACCGTT CCAGATCCCGTCAGGTTCGATGATGCCGACTCTGTTAATTGGTGATTTTATT CTGGTAGAGAAGTTTGCTTATGGCATTAAAGATCCTATCTACCAGAAAACG CTGATCGAAACCGGTCATCCGAAACGCGGCGATATCGTGGTCTTTAAATAT CCGGAAGATCCAAAGCTTGATTACATCAAGCGCGCGGTGGGTTTACCGGG CGATAAAGTCACTTACGATCCGGTCTCAAAAGAGCTGACGATTCAACCGG GATGCAGTTCCGGCCAGGCGTGTGAAAACGCGCTGCCGGTCACCTACTCA AACGTGGAACCGAGCGATTTCGTTCAGACCTTCTCACGCCGTAATGGTGG GGAAGCGACCAGCGGATTCTTTGAAGTGCCGAAAAACGAAACCAAAGAA AATGGAATTCGTCTTTCCGAGCGTAAAGAGACACTGGGTGATGTGACGCA CCGCATTCTGACAGTGCCGATTGCGCAGGATCAGGTGGGGATGTATTACCA GCAGCCAGGGCAACAACTGGCAACCTGGATTGTTCCTCCGGGACAATACT TCATGATGGGCGACAACCGCGACAACAGCGCGGACAGCCGTTACTGGGGC |
| | TTTGTGCCGGAAGCGAATCTGGTCGGTCGGGCAACGGCTATCTGGATGAG CTTCGATAAGCAAGAAGGCGAATGGCCGACTGGTCTGCGCTTAAGTCGCA TTGGCGGCATCCATTAA |
| 81 | TTGAAATCAGAAAATGTTTCGAAGAAAAAGTCAATATTAGAATGGGCAAA AGCAATTGTGATTGCTGTCGTTCTTGCTTTGCTCATCCGCAACTTTATTTTT GCGCCGTATGTCGTTGATGGTGACTCTATGTATCCTACACTTCACAACCGTG AAAGGGTTTTTGTTAATATGACAGTCAAATACATCGGCGAGTTTGATAGAG GAGACATCGTCGTGTTAAACGGAGATGATGTTCACTATGTCAAACGTATTAT CGGCCTTCCCGGCGATACGGTTGAGATGAAAAATGACCAGCTCTATATCAA CGGGAAAAAGGTGGACGAACCTTATTTGGCGGCTAATAAAAAGAGAGCGA AACAGGACGGTTTTGACCATTTGACCGATGATTTCGGCCCGGTTAAAGTGC CTGATAACAAGTATTTTGTGATGGGTGACAATCGTCGCAATTCCATGGACA GCCGTAACGGCCTTGGCCTCTTCACGAAAAAACAAATTGCGGGTACGTCA AAGTTTGTTTTCTACCCGTTTAACGAAATGCGCAAAACAAATTAG |
| 82 | TTGACCGAGGAAAAAAATACGAATACTGAGAAAACGGCGAAGAAAAAAA CCAATACGTACCTGGAATGGGGTAAAGCGATTGTCATCGCTGTTCTGCTGG CTCTCCTGATCCGTCACTTTTTGTTTGAACCGTATTTAGTTGAAGGTTCATC TATGTATCCCACATTACATGACGGAGAAAGGCTGTTTGTGAATAAAACAGT CAACTATATCGGCGAGCTGAAGCGCGGAGATATCGTTATTATCAACGGTGA AACTTCTAAAATCCATTATGTAAAAAGATTGATCGGAAAGCCTGGAGAAAC CGTTCAAATGAAGGATGACACGCTTTATATAAACGGTAAAAAAGTAGCCGA GCCTTACTTGTCTAAAAACAAGAAGGAAGCAGAAAAACTTGGTGTCAGTC TGACAGGAGACTTTGGACCGGTTAAGGTTCCGAAAGGCAAATACTTTGTC ATGGGAGATAACCGGCTGAATTCTATGGACAGCCGAAACGGGCTGGGACT GATCGCGGAAGATCGAATTGTCGGCACATCGAAGTTTGTCTTTTTCCCGTTT AACGAAATGCGTCAAACAAAATAA |
| 83 | GTGACTGATTTTTCTAGTGCTTCAAATGCTGACGATTCCACGCAGGACGGT CGTCCTGGTCGACGTGCTGGAAAGTCTAAGAAGGAATCGAAGCCAACTCC GTGGTACATCGAAATTCCAGTGGTTGTGGTTCTGACCCTCGCGCTGATTTT |
| | CGTGCTCCAGACGTTTGTCGGACGCATGTACATGATTCCGAGTGGTTCGAT GGAACCTACTTTGCACGGATGTGAGGGCTGCACGGGTGACCGCATCCTGG TGGAGAAGGTTTCTTACTACTTCACGGATCCAGAGCCGGGCGATGTTGTGG TGTTCAAGGGTACTGATTCCTGGAACGTTGGATTCACTACGCAGCGTTCCG ATAATTCGGTGATCCGCGGCCTGCAGAACCTGGGTTCTTACGTGGGTCTTG TCGCACCTGATGAAAATGACCTGGTCAAGCGCATTATCGCCACCGGCGGTC AGACTGTTTCGTGCCAAGCCGGTGATCCTGGAATCATGGTTGACGGCAAG GAAGTCGATGACAGCTACACGCTGCAACCTGCGCAATTCCCCATCGATGA GACCTCCGGTTCCACCGAATGCGGCGGCAACTATTTCGGCCCCATCACCGT GCCTGACGGCAACTACTTCATGATGGGTGACAACCGCACCAACTCCATGG ATTCCCGCTACCACCTGGGCGATCAGTACCAAGGAACCATCCCTGAGGAA AACATCAAGGGCAAAGTTCAAGCAATTATCCTGCCATTTAGCCGAATCGGT GGCGTCGACGACCCTGCCATCAAAGGCTAG |
| 84 | ATGATGAAATTTTTAAAAGAATGGGGATTATTTATCTTTATAATTGCCGCTGT CCTTCTCTCGCGCGTCTTTATTTGGTCACTAGTTGTCGTTGATGGCCATTCA ATGGACCCTACTTTAGCCGATAAAGAAAGACTTGTAATTGTTAGAACGACA AAAATTAATCGTTTTGATATTGTAGTTGCTAAAGAAAACGCGGCTGATGGTT CAACCAAAGATATTGTCAAACGTGTCGTTGGGATGCCTGGGGACACTATAA AATTCGACCATGACCAACTTACTATCAATAATAAGGTTTATCCAGAAAACTA TCTCAAAGACTATCAAAAACAATTGGCTGATGGTCAATTGGAAAAAACTTA CGGGAACTATCCTTTGACAAAAGCATTAACTGATCAAAATCGTAGTTTATTT GTAAGCTTAGCTCAGAGCACCAAAGCTTTTACAACGGATAGTACTGGTAAT CCAACCTTTACAGTCAAAGTCCCTGACGGACAATACTTCTTGATGGGAGAT AATCGTGTTGTGTCTCAAGATAGCCGAGCAGTTGGAAGTTTCAAACGTTCA GCGATTATTGGTGAAGCCAAATTACGAGTTTGGCCACTCAATAAAATTTCTT TCTTTTAA |
| 85 | GATTCAAATGGCAATCAAGAAATCAACGGAAAAGAAAAATTAAGCGTTAA TGATAGTAAGTTGAAGGATTTTGGTAAGACAGTTCCGGTTGGTATTGACGA AGAAAATGGTATGATAAAAGTAAGCTTTATGCTTACTGCGCAATTCTATGAA |
| | ATTAAACCTACCAAAGAAAATGAGCAATATATTGGTATGCTTCGCCAAGCT GTTAAAAATGAATCTCCAGTCCACATTTTTTTAAAACCAAACTCAAATGAA ATCGGGAAAGTTGAGAGCGCAAGTCCAGAGGACGTAAGATATTTCAAGAC GATTTTAACTAAAGAAGTAAAAGGACAGACAAACAAATTGGCGAGTGTCA TTCCAGATGTAGCTACATTAAACTCATTGTTCAATCAAATTAAAAACCAATC TTGTGGTACTTCTACTGCTTCAAGTCCATGCATTACTTTTCGATATCCAGTA GACGGGTGTTATGCACGTGCCCATAAAATGCGTCAAATATTGATGAATAATG GCTATGATTGTGAAAAACAATTTGTGTATGGTAACTTAAAAGCATCTACTGG AACCTGTTGTGTTGCATGGAGCTATCATGTTGCAATTTTGGTTAGTTACAAA AATGCTTCTGGAGTGACGGAAAAACGTATTATTGATCCATCACTTTTTTCAA GCGGTCCTGTGACAGATACAGCCTGGAGAAATGCTTGCGTTAATACATCTT GCGGCTCTGCTTCAGTTAGTTCATATGCTAATACCGCAGGAAATGTTTACTA TCGATCTCCCAGTAATTCTTACTTGTATGATAACAATCTTATCAATACAAACT GTGTCTTGACTAAGTTTTCATTACTTTCAGGATGTTCTCCTTCACCTGCCCC TGATGTCTCAAGTTGTGGATTTCATCATCACCATCATCATTAA |
| 86 | CGTCGTGCCACCGCAGATTCAAATGGCAATCAAGAAATCAAC |
| 87 | ATCATGCCTTCTCCTTTAATGATGATGGTGATGATGAAATCC |
| 88 | AGGAGAAGGCATGATGAAATTTTTAAAAG |
| 89 | TGCGGTGGCACGACGAGGAGTC |
| 90 | CTTTTGCTCAATATTATCTTAGCTG |
| 91 | AACTGTTCTTTTTTAATTTTTCCTC |
| 92 | TAAAAAAGAACAGTTGCATGCGGTACCACTAGTATGCCA |
| 93 | AATATTGAGCAAAAGAGATCTGGAGCTGTAATATAAAAACC |

### Industrial Applicability

According to the present invention, even bacteria without an endogenous Tat system secretion apparatus can express a heterologous protein using a Tat system. Therefore, the present invention is extremely useful industrially.

## Claims

1. A bacterium selected from the group consisting of the genus Bifidobacterium, the genus Lactococcus, the genus Lactobacillus, the genus Limosilactobacillus, and the genus Staphylococcus, which has been modified to express a heterologous Tat protein, with an ability to produce and secrete a heterologous protein.

2. The bacterium according to claim 1, wherein the bacterium is a bacterial strain that does not have an endogenous Tat system secretion apparatus.

3. The bacterium according to claim 1 or 2, which has an ability to extracellularly secrete the heterologous protein through a heterologous Tat protein.

4. The bacterium according to any one of claims 1 to 3, wherein the bacterium is selected from the group consisting of Lactococcus Lactis, Bifidobacterium longum, Lactobacillus reuteri (also called Limosilactobacillus reuteri), and Staphylococcus epidermidis.

5. The bacterium according to any one of claims 1 to 4, containing a genetic construct that includes, in a 5' to 3' direction, a nucleic acid sequence encoding a Tat system-dependent signal peptide and a nucleic acid sequence encoding a heterologous protein.

6. The bacterium according to claim 5, wherein the genetic construct further includes a nucleic acid sequence encoding a heterologous Tat protein.

7. The bacterium according to any one of claims 1 to 6, wherein the heterologous Tat protein is a Tat protein derived from bacteria selected from the genus Corynebacterium, the genus Bacillus, and the genus Bifidobacterium.

8. The bacterium according to any one of claims 1 to 7, wherein the heterologous Tat protein includes at least one selected from TatA, TatB, and TatC.

9. The bacterium according to any one of claims 1 to 8, wherein the heterologous Tat protein includes TatA, TatB and TatC.

10. The bacterium according to any one of claims 1 to 9, wherein the heterologous protein includes at least one selected from FGF2 and FGF1.

11. The bacterium according to any one of claims 5 to 10, wherein the genetic construct further includes a promoter derived from bacteria selected from the group consisting of the genus Bifidobacterium, the genus Lactococcus, the genus Lactobacillus, the genus Limosilactobacillus, and the genus Staphylococcus.

12. The bacterium according to any one of claims 5 to 11, wherein the genetic construct further includes a nucleic acid sequence encoding a signal peptidase.

13. The bacterium according to any one of claims 5 to 12, further containing a recombinant vector into which the genetic construct has been incorporated.

14. A method for producing a heterologous protein, including: culturing the bacterium according to any one of claims 1 to 13 to produce and secrete a heterologous protein.

15. A pharmaceutical composition containing the bacterium according to any one of claims 1 to 13.

16. The pharmaceutical composition according to claim 15 for treating or preventing a disease or condition selected from cancer, lower limb ischemic diseases, rashes or intraoral mucositis accompanying cancer treatment, ichthyosis, cavities, irritable bowel syndrome, obesity, injuries, and surgical scars.
